# EUROPEAN PATENT APPLICATION

(11) **EP 1 321 525 A2**
(43) Date of publication of application: **25.06.2003**
(21) Application number: 02028930.2
(22) Date of filing: 21.01.1997
(51) Int. Cl.: C12N 15/82

(54) **Fruit ripening-related genes**

(30) Priority: 23.01.1996 GB 9601330; 09.09.1996 GB 9618742
(62) Divisional of application: 97900698.8
(71) Applicant: Horticulture Research International, Wellesbourne, Warwick CV35 9EF (GB)
(72) Inventor: Manning, Kenneth, c/oHorticulture Research Intern., Warwick, CV35 9EF (GB)
(74) Representative: Ruffles, Graham Keith

(57) **Abstract**

A vector for use in the genetic transformation of strawberry cells comprises a promoter sequence, a regulation sequence and a transcription termination sequence, in which the regulation sequence comprises the coding region, or a fragment of at least 10 bases thereof, of a strawberry protein selected from O-methyl transferase, acyl carrier protein (ACP), elongation factor, auxin-induced gene, cysteine(thiol) proteinase, cellulase, starch phosphorylase, pyruvate decarboxylase, chalcone reductase, protein kinase, auxin-related gene, sucrose transporter, meristem pattern gene, or selected from a strawberry protein with homology to transcribed sequence accession number T45086, transcribed sequence accession number L36159 or transcribed sequence accession number T45902, or selected from a strawberry protein of unknown homology encoded by one of the StrawRipe sequences A to K.

## Description

This invention relates generally to the modification of a plant phenotype by the regulation of plant gene expression. More specifically it relates to the control of fruit ripening by control of one or more than one gene which is known to be implicated in that process.

### BACKGROUND OF THE INVENTION

Two principal methods for the control of expression are known. These are referred to in the art as "antisense downregulation" and "sense downregulation" or "cosuppression". Both of these methods lead to an inhibition of expression of the target gene. Overexpression is achieved by insertion of one or more than one extra copies of the selected gene. Other lesser used methods involve modification of the genetic control elements, the promoter and control sequences, to achieve greater or lesser expression of an inserted gene.

In antisense downregulation, a DNA which is complementary to all or part of the target gene is inserted into the genome in reverse orientation and without its translation initiation signal. The simplest theory is that such an antisense gene, which is transcribable but not translatable, produces mRNA which is complementary in sequence to mRNA product transcribed from the endogenous gene: that antisense mRNA then binds with the naturally produced "sense" mRNA to form a duplex which inhibits translation of the natural mRNA to protein. It is not necessary that the inserted antisense gene be equal in length to the endogenous gene sequence: a fragment is sufficient. The size of the fragment does not appear to be particularly important. Fragments as small as 40 or so nucleotides have been reported to be effective. Generally somewhere in the region of 50 nucleotides is accepted as sufficient to obtain the inhibitory effect. However, it has to be said that fewer nucleotides may very well work: a greater number, up to the equivalent of full length, will certainly work. It is usual simply to use a fragment length for which there is a convenient restriction enzyme cleavage site somewhere downstream of fifty nucleotides. The fact that only a fragment of the gene is required means that not all of the gene need be sequenced. It also means that commonly a cDNA will suffice, obviating the need to isolate the full genomic sequence.

The antisense fragment does not have to be precisely the same as the endogenous complementary strand of the target gene. There simply has to be sufficient sequence similarity to achieve inhibition of the target gene. This is an important feature of antisense technology as it permits the use of a sequence which has been derived from one plant species to be effective in another and obviates the need to construct antisense vectors for each individual species of interest. Although sequences isolated from one species may be effective in another, it is not infrequent to find exceptions where the degree of sequence similarity between one species and the other is insufficient for the effect to be obtained. In such cases, it may be necessary to isolate the species-specific homologue.

Antisense downregulation technology is well-established in the art. It is the subject of several textbooks and many hundreds of journal publications. The principal patent reference is European Patent No. 240,208 in the name of Calgene Inc. There is no reason to doubt the operability of antisense technology. It is well-established, used routinely in laboratories around the world and products in which it is used are on the market.

Both overexpression and downregulation are achieved by "sense" technology. If a full length copy of the target gene is inserted into the genome then a range of phenotypes is obtained, some overexpressing the target gene, some underexpressing. A population of plants produced by this method may then be screened and individual phenotypes isolated. As with antisense, the inserted sequence is lacking in a translation initiation signal. Another similarity with antisense is that the inserted sequence need not be a full length copy. Indeed, it has been found that the distribution of over-and under-expressing phenotypes is skewed in favour of underexpression and this is advantageous when gene inhibition is the desired effect. For overexpression, it is preferable that the inserted copy gene retain its translation initiation codon. The principal patent reference on cosuppression is European Patent 465,572 in the name of DNA Plant Technology Inc. There is no reason to doubt the operability of sense/cosuppression technology. It is well established, used routinely in laboratories around the world and products in which it is used are on the market.

Sense and antisense gene regulation is reviewed by Bird and Ray in Biotechnology and Genetic Engineering Reviews 9: 207-227 (1991). The use of these techniques to control selected genes in tomato has been described by Gray et al., Plant Molecular Biology, 19: 69-87 (1992).

Gene control by any of the methods described requires insertion of the sense or antisense sequence, with appropriate promoters and termination sequences containing polyadenylation signals, into the genome of the target plant species by transformation, follow by regeneration of the transformants into whole plants. It is probably fair to say that transformation methods exist for most plant species or can be obtained by adaptation of available methods.

For dicotyledonous plants the most widely used method is *Agrobacierium*-mediated transformation. This is the best known, most widely studied and, therefore, best understood of all transformation methods. The rhizobacterium *Agrobacterium* *tumefaciens*, or the related *Agrobacterium* rhizogenes, contain certain plasmids which, in nature, cause the formation of disease symptoms, crown gall or hairy root tumours, in plants which are infected by the bacterium. Part of the mechanism employed by *Agrobacterium* in pathogenesis is that a section of plasmid DNA which is bounded by right and left border regions is transferred stably into the genome of the infected plant. Therefore, if foreign DNA is inserted into the so-called "transfer" region (T-region) in substitution for the genes normally present therein, that foreign gene will be transferred into the plant genome. There are many hundreds of references in the journal literature, in textbooks and in patents and the methodology is well-established. Agrobacterium-mediated transformation of the cultivated strawberry (*Fragaria* x *ananassa* Duch. is described in Plant Science, 69, 79-94 (1990).

The effectiveness of *Agrobacterium* is restricted to the host range of the microorganism and is thus restricted more or less to dicotyledonous plant species. In general monocotyledonous species, which include the important cereal crops, are not amenable to transformation by the *Agrobacierium* method. Various methods for the direct insertion of DNA into the nucleus of monocotyledon cells are known.

In the ballistic method, microparticles of dense material, usually gold or tungsten, are fired at high velocity at the target cells where they penetrate the cells, opening an aperture in the cell wall through which DNA may enter. The DNA may be coated on to the microparticles or may be added to the culture medium.

In microinjection, the DNA is inserted by injection into individual cells via an ultrafine hollow needle.

Another method, applicable to both monocotyledons and dicotyledons, involves creating a suspension of the target cells in a liquid, adding microscopic needle-like material, such as silicon carbide or silicon nitride " whiskers", and agitating so that the cells and whiskers collide and DNA present in the liquid enters the cell.

In summary, then, the requirements for both sense and antisense technology are known and the methods by which the required sequences may be introduced are known. What remains, then is to identify genes whose regulation will be expected to have a desired effect, isolate them or isolate a fragment of sufficiently effective length, construct a chimeric gene in which the effective fragment is inserted between promoter and termination signals, and insert the construct into cells of the target plant species by transformation. Whole plants may then be regenerated from the transformed cells.

This invention is concerned with the control of ripening in fruit, and the particular interest here is in strawberries.

The interest in controlling the ripening process is to improve the flavour and/or texture of the fruit, both characters being largely affected by the ripening process. Sugars are the most important soluble component of the flavour. Some 99% of the soluble sugars in strawberry are accounted for by sucrose, glucose and fructose, the amount of these sugars being affected by the season but their relative proportions are largely unaffected.

There is little information in the literature on the metabolic pathways involved in the synthesis of sugars in strawberry. It is known, however that sugars are synthesised during the ripening of the fruit.

The changes in gene expression during strawberry fruit ripening and their regulation by auxin have been described in Planta 194: 62-68 (1994)

### OBJECT OF THE INVENTION

An object of the present invention is to provide DNA sequences enabling the construction of vectors suitable for genetic transformation of strawberry plants, with a view to control of the ripening process in strawberry fruit.

### SUMMARY OF THE INVENTION

According to the present invention there is provided a vector for use in the genetic transformation of strawberry cells, comprising a promoter sequence, a regulation sequence and a transcription termination sequence, in which the regulation sequence comprises the coding region, or a fragment of at least 10 bases thereof, of a strawberry protein selected from O-methyl transferase, acyl carrier protein (ACP), elongation factor, auxin-induced gene, cysteine(thiol) proteinase, cellulase, starch phosphorylase, pyruvate decarboxylase, chalcone reductase, protein kinase, auxin-related gene, sucrose transporter, meristem pattern gene, or selected from a strawberry protein with homology to transcribed sequence accession number T45086, transcribed sequence accession number L36159 or transcribed sequence accession number T45902, or selected from a strawberry protein of unknown homology encoded by one of the StrawRipe sequences A to K.

The gene regulation sequence may be in the same or antisense orientation as the endogenous target gene. It may also be of partial or full sequence length. The invention further contemplates the overexpression of one or more of the genes by inserting into the strawberry genome one or more than one extra copy thereof.

The invention also provides a gene regulation sequence which comprises the coding region, or a fragment of at least 10 bases thereof, of a strawberry protein selected from 0-methyl transferase, acyl carrier protein (ACP), elongation factor, auxin-induced gene, cysteine(thiol) proteinase, cellulase, starch phosphorylase, pyruvate decarboxylase, chalcone reductase, protein kinase, auxin-related gene, sucrose transporter, meristem pattern gene, or selected from a strawberry protein with homology to transcribed sequence accession number T45086, transcribed sequence accession number L36159 or transcribed sequence accession number T45902, or selected from a strawberry protein of unknown homology encoded by one of the StrawRipe sequences A to K.

The sequences of this invention can also be used as probes for isolation of similar sequences from the strawberry genome.

The invention also provides a strawberry plant and propagating material thereof which contains a vector of this invention.

Further according to the invention, there is provided a method for altering the phenotype of strawberry plants, with the aim of controlling the ripening of strawberry fruit, comprising inserting into the genome of the cell of a strawberry plant a gene regulation vector of this invention.

In this way, the invention further provides genetically modified strawberry plants, propagation material and strawberry fruit.

### PREFERRED EMBODIMENTS

In the present invention, the regulation sequence comprises the coding region, or a fragment of at least 10 bases thereof, of a strawberry protein. The strawberry protein is selected from 0-methyl transferase, acyl carrier protein (ACP), elongation factor, auxin-induced gene, cysteine(thiol) proteinase, cellulase, starch phosphorylase, pyruvate decarboxylase, chalcone reductase, protein kinase, auxin-related gene, sucrose transporter, meristem pattern gene, or selected from a strawberry protein with homology to transcribed sequence accession number T45086, transcribed sequence accession number L36159 or transcribed sequence accession number T45902, or selected from a strawberry protein of unknown homology encoded by one of the StrawRipe sequences A to K.

Examples of suitable regulation sequences are SEQ ID NO:1: to SEQ ID NO:27:, also referred to herein as Sequences 1 to 27. Related sequences taken from the priority documents of the present PCT application are given in SEQ ID NO:28: to SEQ ID NO:38:.

The gene regulation sequences of the invention may be synthesised from the sequence information given or may be isolated from a library. To assist isolation Zeneca Limited have deposited with the National Collection of Industrial & Marine Bacteria, St. Machar Drive, Aberdeen, UK, a cDNA library of strawberry ripening genes. The library was deposited on 15th November 1994 under the Budapest Treaty and has the Accession Number NCIMB 40690.

Thus, this invention is based on the identification of genes which encode proteins implicated in strawberry ripening-related processes. DNA sequences which encode these proteins have been cloned and some have been characterised. The DNA sequences may be used to modify plants with the goal of modifying the ripening characteristics of fruit.

By virtue of this invention strawberry plants can be generated which, amongst other phenotypic modifications, may have one or more of the following fruit characteristics:
improved resistance to damage during harvest, packaging and transportation due to slowing of the ripening and over-ripening processes;
longer shelf life and better storage characteristics due to reduced activity of degradative pathways (e.g. cell wall hydrolysis),
improved processing characteristics due to changed activity of proteins/enzymes contributing to factors such as: viscosity, solids, pH, elasticity;
improved flavour and aroma at the point of sale due to modification of the sugar/acid balance and other flavour and aroma components responsible for characteristics of the ripe fruit;
modified colour due to changes in activity of enzymes involved in the pathways of pigment biosynthesis (e.g. lycopene, β-carotene, chalcones and anthocyanins), increased resistance to post-harvest pathogens such as fungi.

The activity of the ripening-related proteins may be either increased or reduced depending on the characteristics desired for the modified plant part (fruit, leaf, flower, etc). The levels of protein may be increased; for example, by incorporation of additional genes. The additional genes may be designed to give either the same or different spatial and temporal patterns of expression in the fruit. "Antisense" or "partial sense" or other techniques may be used to reduce the expression of ripening-related protein.

The activity of each ripening-related protein or enzyme may be modified either individually or in combination with modification of the activity of one or more other ripening-related proteins/enzymes. In addition, the activities of the ripening-related proteins/enzymes may be modified in combination with modification of the activity of other enzymes involved in fruit ripening or related processes.

DNA constructs according to the invention may comprise a base sequence at least 10 bases (preferably at least 35 bases) in length for transcription into RNA. There is no theoretical upper limit to the base sequence - it may be as long as the relevant mRNA produced by the cell - but for convenience it will generally be found suitable to use sequences between 100 and 1000 bases in length. The preparation of such constructs is described in more detail below.

As a source of the DNA base sequence for transcription, a suitable cDNA or genomic DNA or synthetic polynucleotide may be used. The isolation of suitable ripening-related sequences is described above; it is convenient to use DNA sequences derived from the ripening-related clones deposited at NCIMB in Aberdeen. Sequences coding for the whole, or substantially the whole, of the appropriate ripening-related protein may thus be obtained. Suitable lengths of this DNA sequence may be cut out for use by means of restriction enzymes. When using genomic DNA as the source of a base sequence for transcription it is possible to use either intron or exon regions or a combination of both.

In a variation of the vector of this invention the regulation sequence varies from Sequences I to 27 but retains sufficient similarity to be effective in gene regulation. Thus, the regulatory gene may be a homologue of a gene of Sequence 1 to 27 which has been obtained from a strawberry plant.

To obtain constructs suitable for expression of the appropriate ripening-related sequence in plant cells, the cDNA sequence as found in one of the strawberry plasmids or the gene sequence as found in the chromosome of the strawberry plant may be used. Recombinant DNA constructs may be made using standard techniques. For example, the DNA sequence for transcription may be obtained by treating a vector containing said sequence with restriction enzymes to cut out the appropriate segment. The DNA sequence for transcription may also be generated by annealing and ligating synthetic oligonucleotides or by using synthetic oligonucleotides in a polymerase chain reaction (PCR) to give suitable restriction sites at each end. The DNA sequence is then cloned into a vector containing upstream promoter and downstream terminator sequences. If antisense DNA is required, the cloning is carried out so that the cut DNA sequence is inverted with respect to its orientation in the strand from which it was cut.

Promoters suitable for use in constructs of the invention may be any suitable promoters which are known to be effective in driving expression of foreign genes in plants, for example the promoters may be those which are isolatable from the genomic version of the cDNAs of the invention.

In a construct expressing antisense RNA, the strand that was formerly the template strand becomes the coding strand, and vice versa. The construct will thus encode RNA in a base sequence which is complementary to part or all of the sequence of the ripening-related RNA. Thus the two RNA strands are complementary not only in their base sequence but also in their orientations (5' to 3')

In a construct expressing sense RNA, the template and coding strands retain the assignments and orientations of the original plant gene. Constructs expressing sense RNA encode RNA with a base sequence which is homologous to part or all of the sequence of the mRNA. In constructs which express the functional ripening-related protein, the whole of the coding region of the gene is linked to transcriptional control sequences capable of expression in plants.

For example, constructs according to the present invention may be made as follows. A suitable vector containing the desired base sequence for transcription is treated with restriction enzymes to cut the sequence out. The DNA strand so obtained is cloned (if desired, in reverse orientation) into a second vector containing the desired promoter sequence and the desired terminator sequence. Suitable promoters include the 35S cauliflower mosaic virus promoter, the polyubiquitin promoter and the tomato polygalacturonase gene promoter sequence (Bird et al, 1988, Plant Molecular Biology, 11:651-662) or other developmentally regulated fruit promoters. Suitable terminator sequences include that of the *Agrobacterium tumefaciens* nopaline synthase gene (the nos 3' end).

The transcriptional initiation region (or promoter) operative in plants may be a constitutive promoter (such as the 35S cauliflower mosaic virus promoter) or an inducible or developmentally regulated promoter (such as fruit-specific promoters), as circumstances require. For example, it may be desirable to modify ripening-related protein activity only during fruit development and/or ripening. Use of a constitutive promoter will tend to affect ripening-related protein levels and functions in all parts of the plant, while use of a tissue specific promoter allows more selective control of gene expression and affected functions. Thus in applying the invention it may be found convenient to use a promoter that will give expression during fruit development and/or ripening. Thus the antisense or sense RNA is produced only in the organ in which its action is required and/or only at the time required. Fruit development and/or ripening-specific promoters that could be used include the ripening-enhanced polygacturonase promoter (PCT/WO 92/08798), the E8 promoter (Diekman & Fischer, 1988, EMBO, 7:3315-3320), the fruit specific 2AII promoter (Pear et al, 1989, Plant Molecular Biology, 13:639-651), the histidine decarboxylase promoter (HDC, Sibia) and the phytoene synthase promoter.

Ripening-related protein or enzyme activity (and hence ripening-related processes and fruit ripening characteristics) may be modified to a greater or lesser extent by controlling the degree of the appropriate ripening-related protein's sense or antisense mRNA production in the plant cells. This may be done by suitable choice of promoter sequences, or by selecting the number of copies or the site of integration of the DNA sequences that are introduced into the plant genome. For example, the DNA construct may include more than one DNA sequence encoding the ripening-related protein or more than one recombinant construct may be transformed into each plant cell.

The activity of each ripening-related protein may be separately modified by transformation with a suitable DNA construct comprising a ripening-related sequence. In addition, the activity of two or more ripening-related proteins may be simultaneously modified by transforming a cell with two or more separate constructs. Alternatively, a plant cell may be transformed with a single DNA construct comprising both a first ripening-related sequence and a second ripening-related sequence.

It is also possible to modify the activity of the ripening-related protein(s) while also modifying the activity of one or more other enzymes. The other enzymes may be involved in cell metabolism or in fruit development and ripening. Cell wall metabolising enzymes that may be modified in combination with a ripening-related protein include but are not limited to: pectin esterase, polygalacturonase, β-galactanase, ,β-glucanase. Other enzymes involved in fruit development and ripening that may be modified in combination with a ripening-related protein include but are not limited to: ethylene biosynthetic enzymes, carotenoid biosynthetic enzymes including phytoene synthase, carbohydrate metabolism enzymes including invertase.

Several methods are available for modification of the activity of the ripening-related protein(s) in combination with other enzymes. For example, a first plant may be individually transformed with a ripening-related gene construct and then crossed with a second plant which has been individually transformed with a construct encoding another enzyme. As a further example, plants may be either consecutively or co-transformed with ripening-related constructs and with appropriate constructs for modification of the activity of the other enzyme(s). An alternative example is plant transformation with a ripening-related construct which itself contains an additional gene for modification of the activity of the other enzyme(s). The ripening-related gene constructs may contain sequences of DNA for regulation of the expression of the other enzyme(s) located adjacent to the ripening-related sequences. These additional sequences may be in either sense or antisense orientation as described in PCT/WO 93/23551 (single construct having distinct DNA regions homologous to different target genes). By using such methods, the benefits of modifying the activity of the ripening-related proteins may be combined with the benefits of modifying the activity of other enzymes.

A DNA construct of the invention is transformed into a target plant cell. The target plant cell may be part of a whole plant or may be an isolated cell or part of a tissue which may be regenerated into a whole plant. For any particular plant cell, the ripening-related sequence used in the transformation construct may be derived from the same plant species, or may be derived from any other plant species (as there will be sufficient sequence similarity to allow modification of related isoenzyme gene expression).

Transgenic plants and their progeny may be used in standard breeding programmes, resulting in improved plant lines having the desired characteristics For example, fruit-bearing plants expressing a ripening-related construct according to the invention may be incorporated into a breeding programme to alter fruit-ripening characteristics and/or fruit quality. Such altered fruit may be easily derived from elite lines which already possess a range of advantageous traits after a substantial breeding programme; these elite lines may be further improved by modifying the expression of a single targeted ripening-related protein/enzyme to give the fruit a specific desired property.

By transforming plants with DNA constructs according to the invention, it is possible to produce plants having an altered (increased or reduced) level of expression of one or more ripening-related proteins, resulting from the presence in the plant genome of DNA capable of generating sense or antisense RNA homologous or complementary to the RNA that generates such ripening-related proteins. For fruit-bearing plants, fruit may be obtained by growing and cropping using conventional methods. Seeds may be obtained from such fruit by conventional methods (for example, tomato seeds are separated from the pulp of the ripe fruit and dried, following which they may be stored for one or more seasons). Fertile seed derived from the genetically modified fruit may be grown to produce further similar modified plants and fruit.

The fruit derived from genetically modified plants and their progeny may be sold for immediate consumption, raw or cooked, or processed by canning or conversion to soup, sauce or paste. Equally, they may be used to provide seeds according to the invention.

The genetically modified plants (transformed plants and their progeny) may be heterozygous for the ripening-related DNA constructs. The seeds obtained from self fertilisation of such plants are a population in which the DNA constructs behave like single Mendelian genes and are distributed according to Mendelian principles: e.g., where such a plant contains only one copy of the construct, 25% of the seeds contain two copies of the construct, 50% contain one copy and 25% contain no copy at all. Thus not all the offspring of selfed plants produce fruit and seeds according to the present invention, and those which do may themselves be either heterozygous or homozygous for the defining trait. It is convenient to maintain a stock of seed which is homozygous for the ripening-related DNA construct. All crosses of such seed stock will contain at least one copy of the construct, and self-fertilized progeny will contain two copies, i.e. be homozygous in respect of the character. Such homozygous seed stock may be conventionally used as one parent in F1 crosses to produce heterozygous seed for marketing. Such seed, and fruit derived from it, form further aspects of our invention. We further provide a method of producing FI hybrid plants expressing a ripening-related DNA sequence which comprises crossing two parent lines, at least one of which is homozygous for a ripening-related DNA construct. A process of producing F1 hybrid seed comprises producing a plant capable of bearing genetically modified fruit homozygous for a ripening-related DNA construct, crossing such a plant with a second homozygous variety, and recovering Fl hybrid seed. It is possible according to our invention to transform two or more plants with different ripening-related DNA constructs and to cross the progeny of the resulting lines, so as to obtain seed of plants which contain two or more constructs leading to reduced expression of two or more fruit-ripening-related proteins.

### EXAMPLES OF THE INVENTION

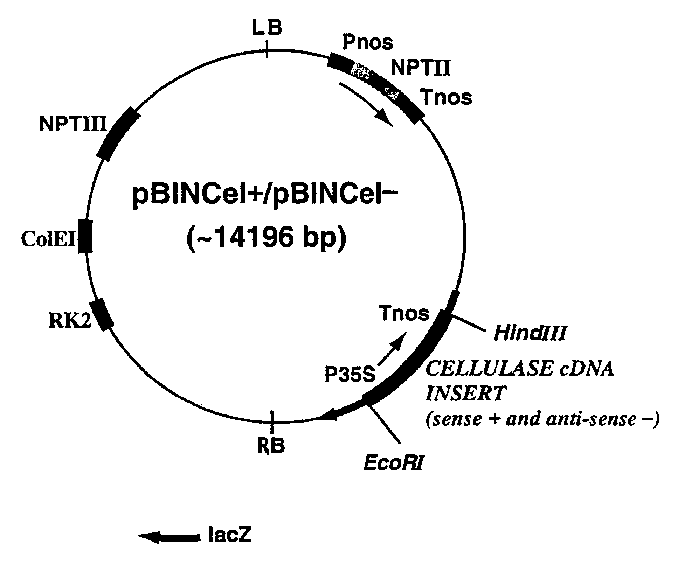

The invention will now be described, by way of illustration, by the following Examples. In the Examples, reference is made to Figure 1.

### THE DRAWING

Figure 1 is a diagrammatic map of plasmid pBINCEL.

### EXAMPLE 1

### Construction of a cDNA library of ripening genes

### 1.1 Isolation of messenger RNA

Total RNA was isolated from ripe fruit tissue (the receptacle with the achenes removed) of strawberry (*Fragaria x ananassa* Duch. cv. Brighton) as described by Manning K. Analytical Biochemistry 195, 45-50 (1991). Messenger RNA was isolated from total RNA by oligo(dT)-cellulose chromatography according to Bantle et al., Analytical Biochemistry 72, 413-427 (1976).

### 1.2 Synthesis of cDNA

The first and second strands of the cDNAs were synthesised from messenger RNAs using a commercial cDNA synthesis kit (RPN.1256Y: Amersham Life Sciences. Amersham, Bucks., UK), priming the first strand cDNA synthesis with oligo-dT.

### 1.3 Cloning into vector

Double stranded cDNAs were cloned into the λgt10 vector using the BRL cloning system (8287SA: Bethseda Research Laboratories, Paisley, Renfrewshire, UK) essentially as follows. Internal EcoRI sites of the cDNAs were methylated using EcoRI methylase. The DNA termini were repaired with T4 DNA polymerase and phosphorylated EcoRI linkers ligated to the cDNA with T4 ligase. Excess linkers were digested and removed by column chromatography on DEAE-Sephadex. The purified double stranded cDNAs with EcoRI termini were ligated into λgt10 vector DNA digested with EcoRI and dephosphorylated. Vector DNA was then packaged using an *in vitro* packaging extract (Promega Corporation, Southampton, UK). Recombinant bacteriophage were mixed with plating bacteria (E. coli C600 hflA 150) as described in the BRL protocol to determine titre, for library screening and subsequent amplification.

### 1.4 Screening of the cDNA library from ripe strawberry

The unamplified cDNA library from ripe strawberry was differentially screened using cDNA from fruit receptacle tissue at the ripe and white stages of ripeness. A proportion of the library was plated at low density and duplicate plaque lifts made on to Hybond N nylon filters (Amersham) according to the manufacturer's instructions. One filter was hybridised to ripe cDNA from white fruit and the duplicate filter hybridised to ripe cDNA. Hybridisations were at high stringency using digoxigenin as a non-radioactive label (Boehringer Mannheim, Lewes, Sussex, UK). Plaques hybridising preferentially to ripe cDNA were picked and replated at low density for a second round of selection by differential screening. Single plaques from the second screening were picked and numbered as ripening-enhanced clones.

### 1.5 Characterisation of the ripe cDNA library and ripening-enhanced clones

The ripe cDNA library was prepared with an efficiency of 3.03x 106 plaque-forming units per microgram of cDNA. The size of the cDNA inserts in this library ranged from approximately 0.24 to 6 kbp with a mean insert size of approximately 1.4 kbp.

From the 1343 plaques used in the first screen, 83 putative ripening clones were obtained. Of these, 48 were pure clones with single inserts, the remainder being impure and having multiple inserts.

The 48 clones with single inserts were partially sequenced using the DyeDeoxy (Trade Mark) Terminator Cycle Sequencing Kit (Applied Biosystems, Warrington, Cheshire, UK) with forward and reverse primers specific for the λgt10 vector. Improved sequence data were obtained for clones with multiple inserts and clones with single inserts that did not produce good sequence data by subcloning into the phagemid vector pBK-CMV (Stratagene) vector for sequencing. From the sequenced clones, the following twenty-seven ripening-related clones were selected. Comparison of these sequences with sequences in the EMBL database using GCG ("Winconsin") software has identified homologies for the clones of sequences 1 to 16 listed in the following table 1.

| Sequence ID NO | Homology/Identity | Clone number |
|---|---|---|
| 1 | O-methyl transfcrase | 1 |
| 2 | acyl carrier protein (ACP) | 3 |
| 3 | elongation factor | 33a |
| 4 | auxin-induced gene | 33b |
| 5 | cysteine(thiol) proteinase | 93c |
| 6 | cellulase | 97 |
| 7 | starch phosphorylase | 6ab |
| 8 | pyruvate decarboxylase | 16bc |
| 9 | chalcone reductase | 31c |
| 10 | protein kinase | 75b |
| 11 | auxin-related gene | 61 c |
| 12 | sucrose transporter | 110ab |
| 13 | meristem pattern gene | 26 |
| 14 | transcribed sequence, T45086 | 13 |
| 15 | transcribed sequence, L36159 | 56 |
| 16 | transcribed sequence, T45902 | 61b |
| 17 | StrawRipe A | 10 |
| 18 | StrawRipe B | 40 |
| 19 | StrawRipe C | 48 |
| 20 | StrawRipe D | 54 |
| 21 | StrawRipe E | 62 |
| 22 | StrawRipe F | 81 |
| 23 | StrawRipe G | 90 |
| 24 | StrawRipe H | 92 |
| 25 | StrawRipe I | 99 |
| 26 | StrawRipe J | 106b |
| 27 | StrawRipe K | 106c |

### 1.6 Expression of ripening enhanced clones

RNA was extracted from strawberry fruit during normal development and analysed by Northern blotting using standard procedures. The level of messenger RNA corresponding to the expression of O-methyl transferase, cysteine proteinase, acyl carrier protein and auxin induced gene were monitored in the receptacle at various time points between pollination and the overripe stage, between Day 1 and Day 19, and then at the stages of Turning, Orange, Ripe and Overripe. Messenger RNA for O-methyl transferase appeared at Day 19, through to Overripe and was highest at Orange and Ripe. The messenger RNA for cysteine proteinase was low up to day 19, and then increased between the Turning and Overripe stages. The messenger RNA for Acyl carrier protein was low up to Day 19, and increased for Turning, Orange and Ripe. The messenger RNA for Auxin induced gene appeared around Day 16, and was highest between the Turning and Overripe Stages.

The data provide evidence that O-methyl transferase, cysteine proteinase, acyl carrier protein and auxin induced gene are involved in the ripening process in normal fruit development.

### EXAMPLE 2

### Construction of antisense RNA vectors with the CaMV35S promoter

A vector is constructed using the sequences corresponding to a fragment of one of the sequences 1 to 38, more especially one of the sequences 1 to 27. This fragment is synthesised by the polymerase chain reaction using synthetic primers. The ends of the fragment are made flush with T4 polymerase and it is cloned into a derivative of the pBINPLUS vector (van Engelen *et al*., Transgenic Research 4, 288-290 (1995)) containing the cauliflower mosaic virus (CaMV) 35S promoter-nopaline synthase (nos) 3' terminator cassette inserted into the HindIII/EcoRI site. For example, in this way, the plasmid pBINCEL is obtained which is derived from pBINPLUS and which contains cellulase cDNA in either the sense or antisense orientation. A diagrammatic map of the plasmid pBINCEL is given in Figure 1. In one particular experiment, an antisense extended sequence comprising the cellulase of SEQ ID:6: with the addition of a polyA tail of 17 bases was inserted to give a pBINCEL antisense cellulase vector.

Alternatively a vector is constructed using a restriction fragment obtained from a strawberry ripening-related clone. The fragment is blunt ended with T4 polymerase and is cloned into a derivative of the pBINPLUS vector.

After synthesis of the vector, the structure and orientation of the sequences are confirmed by DNA sequence analysis.

### EXAMPLE 3

### Construction of antisense RNA vectors with a fruit enhanced promoter.

The fragment of the ripening-related cDNA that was described in Example 2 is also cloned into the vector pJR3. pJR3 is a Bin 19 based vector, which permits the expression of the antisense RNA under the control of the tomato polygalacturonase (PG) promoter. This vector includes approximately 5 kb of promoter sequence and 1.8 kb of 3' sequence from the PG promoter separated by a multiple cloning site

After synthesis, vectors with the correct orientation of the ripening-related sequences are identified by DNA sequence analysis.

Alternative fruit enhanced promoters (E8, 2A11 or any strawberry promoter) are substituted for the polygalactonurase promoter in pJR3 or for the CaMV 35S promoter in the modified pBINPLUS vector described in Example 2 to give alternative patterns of expression.

### EXAMPLE 4

### Construction of truncated sense RNA vectors with the CaMV 35S promoter

The fragment of the ripening-related cDNA that was described in Example 2 is also cloned into the vectors described in Example 2 in the sense orientation.

After synthesis, the vectors with the sense orientation of the phytoene synthase sequence are identified by DNA sequence analysis.

### EXAMPLE 5

### Construction of truncated sense RNA vectors with fruit-enhanced promoter.

The fragment of the ripening-related cDNA that was described in Example 3 is, also cloned into the vectors described in Example 3 in the sense orientation.

After synthesis, the vectors with the sense orientation of the ripening-related sequence are identified by DNA sequence analysis.

### EXAMPLE 6

### Construction of an over-expression vector using the CaMV35S promoter

The complete sequence of a ripening-related cDNA containing a full open-reading frame is inserted into the vectors described in Example 2.

### EXAMPLE 7

### Construction of an over-expression vector using a fruit-enhanced promoter

The complete sequence of a ripening-related cDNA containing a full open-reading frame is inserted into the vectors described in Example 3 (pJR3 or alternatives with different promoters).

### EXAMPLE 8

### Generation of transformed plants

Vectors are transferred to *Agrobacterium tumefaciens* EHA105 (a kanamycin sensitive strain of an organism widely available to plant biotechnologists; Hood et al., Transgenic Research 2, 208-218 (1990)) and are used to transform strawberry plants. Strawberry explants infected with *Agrobacterium* are grown on regeneration medium normally containing 100 mg/l kanamycin. After three weeks, the explants are transferred to regeneration medium without kanamycin. At 4 to 6 weeks, putatively transformed shoots are cultured on propagation medium for two weeks and then transformants are selected on medium containing 25 mg/l kanamycin. Regenerated plants containing the transgene are selected and grown to maturity. Ripening fruit are analyzed for modifications to their ripening characteristics.

For example, transformed plants were produced in this way using the pBINCEL antisense cellulase fragment of Example 2. The presence of the transgene in the putative strawberry transformant was verified by PCR using genomic DNA from the transformant as template and primers from the 35S promoter and from the cellulase strand. The PCR products were separated by agarose gel electrophoresis and a fragment of ∼1400 base pairs was obtained that was identical in size to the PCR product obtained using the pBINCEL antisense cellulase vector DNA as template.

The following sequences have been edited to remove vector bases and polyA regions, as appropriate.

## Claims

1. A vector for use in the genetic transformation of strawberry cells, comprising a promoter sequence, a regulation sequence and a transcription termination sequence, in which the regulation sequence comprises the coding region, or a fragment of at least 10 bases thereof, of a strawberry protein which is meristem pattern gene.

2. A vector according to claim 1, wherein the regulation sequence comprises SEQ ID NO: 13 or a fragment thereof with at least 10 bases.

3. A vector according to claim 1 or 2, wherein the regulation sequence is aligned for antisense expression.

4. A vector according to claim 1 or 2, wherein the regulation sequence is aligned for sense expression.

5. A vector according to any preceding claim, wherein the regulation sequence fragment comprises at least 35 bases.

6. A method for genetic modification of a strawberry comprising inserting a vector as claimed in any preceding claim into the genome of a strawberry plant.

7. Propagation material for a strawberry plant which plant is progeny of a strawberry plant which has been modified by a method according to claim 6.

8. Strawberry fruit of a strawberry plant grown from propagating material according to claim 7.

9. Strawberry fruit according to claim 8, with regulated ripening in comparison with unmodified fruit.

10. A gene regulation sequence SEQ ID NO: 13, and fragments thereof with at least 10 bases.


SEQUENCE LISTING

(1) GENERAL INFORMATION

(i) APPLICANT
(A) NAME: Horticulture Research International
(B) STREET: -
(C) CITY: Stratford-upon-Avon
(D) STATE OR PROVINCE: Warwick
(E) COUNTRY: United Kingdom
(F) POSTAL CODE: CV35 9EF

(ii) TITLE OF INVENTION: Fruit Ripening-Related Genes

(iii) NUMBER OF SEQUENCES: 38

(iv) COMPUTER-READABLE FORM:
(A) MEDIUM TYPE: 1.44 MB Diskette
(B) COMPUTER: DELL Pentium
(C) OPERATING SYSTEM: Windows
(D) SOFTWARE: Word

(2) INFORMATION FOR SEQ ID NO:1:

(i) SEQUENCE CHARACTERISTICS:
(A) LENGTH: 549
(B) TYPE: cDNA
(C) STRANDEDNESS: Single
(D) TOPOLOGY: Linear

(ix)	FEATURES
(D)	OTHER INFORMATION:	O-methyl transferase

(xi)	SEQUENCE DESCRIPTION: SEQ ID:1:
CCNCCNNCTC AATNTNNNNC ATCATNTNTN NGGGGGTTGG GGNTCTNGAA 050
GGCAAAAGAT TCGGTCAGGA CAAGGTCCTC GTCGAGAGCT GGTATCATTT 100
GANGGATGCA GTTCTTGATG GTGGGATTCC ATTTAACAAG GNCTATGGCA 150
TGACTGCATT TGATTACCAT GGNAACTGAC CCTAGCATTC AACAAGGTCT 200
TCAACAAGGG AATGGCTGAC CACTCCACCA TTACCATGCA NGTAAAATCC 250
TTGTAGTACT TACAAAGGCT TCGAGGGCCT CAAATCCATC GTTGTATGTC 300
GGTGGCGGNA CCNGAGCTGT GGNGGAACAT NATCGCTTCC CNAGTTNCCC 350
TTCGCATCAA GGGTCATCAN CCTTTCGACT TGCCCTCAAT CTTANTCGAA 400
NGCATTCCTC CNTCAATTAT CCTNNNTGTT TCCANCCANG TTGGGATGNG 450
GGGANAATCT TCTGGCNANN TCTTACCCAA TTNNGGNANN CTTCCATTCT 500
TTCCCATTTN AGTTCNTNTT TTNCTCAACC TAACTTGNCG NTCCNTCGN 549

(2)	INFORMATION FOR SEQ ID NO:2:

(i)	SEQUENCE CHARACTERISTICS:
(A)	LENGTH:	661
(B)	TYPE:	cDNA
(C)	STRANDEDNESS:	Single
(D)	TOPOLOGY:	Linear

(ix)	FEATURES
(D)	OTHER INFORMATION:	Acyl carrier protein

(xi)	SEQUENCE DESCRIPTION:	SEQ ID:2:

GGTTTTAGAA CTATCCTCGA TCGCATCAAT GGCCGCCACC ACAGGAGCTG 050
CTTCTTCGAT CTCACTCCGC TCTCGCCTTC ACCAGAATCT TGCATCGTCC 100
AGGGTCAATG GTCTTAAGCC AGTTTTACTG TCTGGTAATG GAAGAAGTTC 150
TCTTTCTTTC GGGTTACAGA AGCGTTCAGC ACGGCTTCAG ATTTACTGCG 200
CAGCCAAACC AGAGACAATG GACAAGGTGT GCCAGATAGT TAGAAAGCAA 250
CTTGCATTAC CAGATGACTC GGCAGTTTCT GGAGAGTCAA AATTTTCTGC 300
ACTTGGAGCT GATTCTCTTG ATACGGTTGA GATCGTGATG GGACTTGAGG 350
AGGAATTTGG TTTTAGCGTG GAAGAGGAGA GTGCTCAGAG CATTGCAACC 400
GTTCAGGATG CTGCGGATCT TATCGAGAAG CTCATTGAGA AGAACAATGC 450
TTAGAAGAAG AAATGAGAAA ACAAGAGTCA ATCCTAGCCT GCTTTAGATA 500
ATTATTTGGT TGGTAGACTG GTTATGTATG CAGTCATTTT GTGTGAAATT 550
TGAACCTGAT AGTGGCTTGA GTGTTAAATT ATGAATGTAT GGATTTGAGT 600
TTGTGTGGTC AAGCTCCTTT CTTTCCTATA TTTCTGATGA AATAGAGAAT 650
GGCCTTACAA T 661

(2) INFORMATION FOR SEQ ID NO:3:

(i) SEQUENCE CHARACTERISTICS:
(A) LENGTH: 1026
(B) TYPE: cDNA
(C) STRANDEDNESS: Linear
(D) TOPOLOGY: Single

(ix) FEATURES
(D) OTHER INFORMATION: Elongation factor

(xi) SEQUENCE DESCRIPTION: SEQ ID:3:
GGGCCCATGT TGACAAAGCT CAATGTCACT ATGAAGAGTG ATGAAAAAGA 050
ACTTATGGGA AAGGCATTGA TGAAGAGGGT CATGCAGAAC TGGCTTCCAG 100

CCAGCACTGC CCTATTGGAA ATGATGATCT TTCACCTTCC CTCTCCACAC 150
ACAGCTCAAA AGTACCGTGT TGAGAATTTG TACGAGGGTC CCCTGGATGA 200
CCAATATGCT AATGCTATCA GAAACTGTGA TCCAGATGGT CCGCTTATGC 250
TTGTATTGTA TCTAAGATGA TTCCGGCATC TTGACAAGGG TNAGATTCTT 300
TGGTTTTGGG TCGTGTTGTT TGGCTGGTAG GGGTCCCAAA CTGGTTTGGA 350
NGGGTTAAGG AATTATGGGG ACCCAAACTA TTGTTCCTGG GGAAAAGAGG 400
GATCTTTATG TCAAGAATTG TACAGNGGGA CTTGNNATCT TGGATGGGGA 450
AAAGAAACAA NGAAACTGTT GAGGATGTTC CCCTGTGGTA AAAACTTGTN 500
CCCTTGGTTG GTCTGGGAAN AAGTTCAATC CACCCAAGAA TGCTACCTTG 550
ACCAAATGAG AGGGNAACAA GATGCTCCCC CCATTCGTGC AATGAAGTTC 600
TCCTGTCTCA ACCCTGTTGT GCGTGTTGCT GTTCAANCGT AAGGNTGCTT 650
CTTGATCCTT CCCCAAGCTT GTTGAAGGGC TGAAACGTCT GGCTAAGACC 700
CGATCCCTAT GGGTGTCTGT ACCATTGAGG AGTCTGGAGA GCACATCATT 750
GCTGGAGCTG GTGAACTTCA CCTTGAGATC TNCNTGANGG ATCTNCAAGA 800
TGATTTTATG GGTGGAGCGG AAATTGTAAA ATCTGATCCT GTTGTGTCCT 850
NCCGTGAGAC AGTCCTTGAG AAGNCCTNCC GTACTGTGAT GAGCAAGTCT 900
CCCAACAAGC ACAACCGTCT GTACATGGAA GCACNCCCGT TGGAGGAAGG 950
TCTTCCTGAG NCCATTGATG ATGGTCGTAT TGGNCCAAGG GATGATCCTA 1000
AAATCCGCTC AAAGATCTTG NCTGAG 1026

(2) INFORMATION FOR SEQ ID NO:4:

(i) SEQUENCE CHARACTERISTICS:
(A) LENGTH: 957
(B) TYPE: cDNA
(C) STRANDEDNESS: Single
(D) TOPOLOGY: Linear

(ix) FEATURES
(D) OTHER INFORMATION: Auxin-induced mRNA

(xi) SEQUENCE DESCRIPTION: SEQ ID:4:
GGCACCAGTG CTTCATATCT CGCCCTTTGC AGTTTCACAT ATCAAAGTAG 050
CATCTCAAAT CACATCAATG GCAGACGAGG TTGTCTTGTT GGACTTCTGG 100
CCAAGCCCAT TTGGGATGAG GCTGAGGATC GCTCTGGCCG AGAAAGGCGT 150
CAAGTACGAG TACAAGGACG AGGACCTGAG GAACAAGAGC CCGCTGTTGC 200
TTCAGTCGAA CCCGGTTCAC AAGAAGGATC CCGGTTCTCA TTCACAACGG 250
CAAACTGTCT TGCGAGTCTT GTCATTGCTC TTCAAGTACA TTGACGAGGT 300
CTTGGACTTA ACAAAGCCAC TATTGNCCTC CCGACCCCTT ACCTCAGGAT 350
CCCCAGGCCA GGGTCTTGGG CCGACTTCCG NGGACAAAGA AGATNTTTTG 400
ATNTCGGGTA GGNAAGACAA TGGNCAACGA AAGGAGATTG AGCAGGGAGG 450
CAGNAAAGAA GGGATTCTTC GACTGCATTA AGTTGCTAGA AGTGGAGCTT 500
GGTGACAAGC CTTTCTTTGG CGGTGAGACC CTCGGATTTG TGGACGTGAC 550
GCTCGNTCCT TTCTATTCCT GGTTCTCTGT GTATGAGAAA TACGGCAACT 600
TCAGCATTGC GCCAGAGTGC CCAAAGTNCA TGGCTTGGGT TAAGAGGTGT 650
ATGGAGAAGG AGAGTGTGTC AAAGTCTCTT CCTGACCAGG ACAAGGTCTG 700
TGGCTTNGTT GCCGAGATGA NGAAGAAGCT TGGAGTTGAG TAGATGTGAT 750
CAATGTCATN TTGATCATGT CTTTGTTTTA GCCCCAAGAT TCANCCTCGT 800
TTTGGGTTGC TTGTATTTTT CAATAAAATT GGGGGACTTG GACCAAGCCC 850
TCCAATAGTA GGAAGCACTC TTTCNGTGCC TCTTGGTCCN GTTTTTCTTC 900
NGNTAANCCT NTNTGCAGCT AAAATTCACC GNATTNCTGN TTTCCTTNTA 950
TNGCCAA 957

(2) INFORMATION FOR SEQ ID NO:5:

(i) SEQUENCE CHARACTERISTICS:

(A) LENGTH: 518
(B) TYPE: cDNA
(C) STRANDEDNESS: Single
(D) TOPOLOGY: Linear

(ix) FEATURES
(D) OTHER INFORMATION: Cysteine (thiol) proteinase

(xi) SEQUENCE DESCRIPTION: SEQ ID:5:
ATCTCCTCCT CCTCTCTCTC CTTCTCCTCC TCTCCTCCGC CGTCGCCTCC 050
ACCGTAACCG ACGCCGGCGA TCCTCTCATA CGACAAGTCG TACCGGGCGC 100
GGCCGAGGAT GACGAGCTCC TCCACGCGGA GCGTCACTTC TCGAACTTCA 150
AAGCCACGTT CGGAAAGAGC TACGCGAGCC AGGAGGAGCA CGACTACAGG 200
TTCCGGCGTA TTCAAGGNCA ACTCCGCCGG GCGAAGAGGC ACCAGGGGCT 250
TGGACCCCAC CGCCGTGCAC GGTGTCAACG AAATCTCCGA TCTCACTCCC 300
AAGGAGTTTC GNCGGGAATT TCCTCGGGCT TAAGAAGGGG TCGGANTTCG 350
GGTTACCGGC CGACGGTTAA AAAAGGGGCC NGATNCCTNC CGGANGAATT 400
ANCTTCCCCA CCCANTTTTG GNNTTGGGGN GAAAAAAGGN GCCCGNCNAA 450
GNCGGNGGAA NGGNCAAGGG GGAAATNGGG TNNAATTNGG NCNGGTTNAN 500
NGNGGGCCCG NAGAANTT 518

(2) INFORMATION FOR SEQ ID NO:6:

(i) SEQUENCE CHARACTERISTICS:
(A) LENGTH: 1766
(B) TYPE: cDNA
(C) STRANDEDNESS: Single
(D) TOPOLOGY: Linear

(ix) FEATURES
(D) OTHER INFORMATION: Cellulase (endo-(1,4)beta-n-glucanase

(xi) SEQUENCE DESCRIPTION: SEQ ID:6:
GGCAGCAAAA ACGAGAGAGA AAAAAAAATG GCGCGAAATG GCCTTTGCTT 050
ACCGGGAAAT GCTCCCGCAT TTCGCGCAAC ACTCGTCCTC TCGCTGCTCC 100
TGCTTCTCCA GCCAATCCGC GCCGGCCACG ACTACCACGA CGCCCTCCGC 150
AAGAGCATCC TCTTCTTCGA AGGCCAGCGC TCCGGCAAGC TCCCGCCCGA 200
TCAACGCCTC AAATGGCGCC GCGACTCCGC ATTGCACGAC GGCTCCACCG 250
CCGGCGTAGA CTTAACCGGC GGCTACTACG ACGCCGGCGA CAACGTGAAG 300
TTCGGGTTTC CGATGGCGTT CACGACCACT CTGCTGGCGT GGAGCATTAT 350
AGACTTCGGG AGGGTCATGG GGACGGAGCA GAGGAACGCG GTCAAGGCGT 400
TACGGTGGGG GACAGACTAC CTCCTGAAGG CCACGGCGGT TCCTGGCGTC 450
GTCTTCGTCC AAGTCGGCGA CCCATACTCC GATCACAACT GCTGGGAGAA 500
GCCGGAAGAC ATGGACACAC GCCGCACGGT GTACAAAATC GACCACAACA 550
ACCCGGGATC CGACGTGGCA GGCGAAACCG CAGCCGCGCT CGCCGCCGCC 600
TCCATCGTTT TCAGGTCACG TGACCCCGCT TACTCGAGAC TGCTTCTCAA 650
TCGAGCCGTT AAGGTTTTCG AGTTCGCTGA TACCCACCGC GGCGCGTACA 700
GCTCCAGCCT CAAAAACGCC GTGTGCCCTT TTTACTGCGA CGTCAACGGC 750
TTCCAGGATG AGTTACTGTG GGGAGCAGCG TGGTTGCACA AGGCGTCGAG 800
AAGGCGGCAG TACAGAGAAT ACATAGTGAG AAACGAGGTC ATTTTGAGAG 850
CTGGAGATAC CATTAACGAG TTTGGTTGGG ATAACAAGCA TGCTGGGATT 900
AATATTCTCA TTTCTAAGGA AGTGCTTATG GGAAAAGCAG ATTATTTCGA 950
ATCTTTCAAG CAAAATGCAG ATGGATTTAT ATGCTCTGTT TTGCCTGGAC 1000
TTGCCCATAC CCAAGTCCAA TATTCTCCAG GTGGTTTGAT CTTCAAGCCT 1050
GGAGGGAGTA ACATGCAGCA TGTAACTTCG CTATCGTTCC TGCTTTTGAC 1100
TTATTCCAAC TATCTAAGCC ACGCCAATAA GAACGTGCCG TGTGGCATGA 1150
CCTCCGCCTC CCCGGCCTTC CTCAAACAAT TGGCTAAACG CCAGGTGGAT 1200
TACATTTTGG GTGACAATCC ATTAAGAATG TCTTACATGG TTGGATATGG 1250
GCCGCGTTAC CCGCAAANGA TTCACCACCG GGGCAGCTCA CTTCCATCCG 1300

TGCAGGCCCA TCCGGCCCGT ATCGGATGCA AAGCCGGTTC TCATTATTTT 1350
CTGAGTCCGA ATCCAAACCC GAATAAATTA GTCGGGGCTG TTGTGGGCGG 1400
ACCCAATAGC TCGGATGCAT TTCCGGACTC GAGGCCTTAC TTTCAAGAGT 1450
CTGAGCCCAC GACGTACATA AATGCGCCTC TTGTGGGCCT ACTTTCGTAT 1500
TTTGCAGCCC ATTACTAATT CTCGAAGTGT AAACAGTGAT TGAGAATTTG 1550
TTGTGGTGCG CCAATACTCA CCCACCAATC CCCCACACTA CCAATTGTTG 1600
TTACTTTTGG AAAGTTCTAA ATTTAAGAAA TTGTTAAGAA AGAAAATGGC 1650
CCAAGCTTAG TTATGGAATT TAGTCTCAAA AGCCCTACTG TTGTGCTTTT 1700
GAAATGTTCT AGCTGTAACA TAATTTCTAT CAATGAATAA AGAAAATGGG 1750
CCAAGCCTAA ATGTGG 1766

(2) INFORMATION FOR SEQ ID NO:7:

(i) SEQUENCE CHARACTERISTICS:
(A) LENGTH: 585
(B) TYPE: cDNA
(C) STRANDEDNESS: Single
(D) TOPOLOGY: Linear

(ix) FEATURES
(D) OTHER INFORMATION: Starch phosphorylase

(xi) SEQUENCE DESCRIPTION: SEQ ID:7:
AATCCTGGGG GGNTTNCCCA CCCTTAANTT GGCNGNNGAT NTTTTTGATA 50
CTCNTCGGGG GGGCGGAANC CTATGGGGAG AANNGGCAAC CAAAGGNGCC 100
TTTTNTAGGG TTGCCTGGCN TATTTACTGG CCTGGTNCTN AACATGTNCT 150
TTCCTGCGAT ATCCCCTGAT TCTGNGGATA ANCCGTATNA CNCGCCNNTG 200
AGTGAGGCTG ATACCGCTNC ACCGCATCCG ACCGACCGAT CGCAGCGAGT 250
CAGTGAGCGA GGAAGCGGAA GAGCGCCCAA TACGCAAGCC ACCTCTCNCC 300

GCGCGTTGGC CGATTCATTA ATGCAGCTGG CACGACAGGT TTCCCGACTG 350
GAAAGCGGGC AGTGAGCGCA ACGCAATTAA TGTGAGTTAG CTCACTCATT 400
AGNCACCCCA GGCTTTACAC TTTATGCTTC CGGCTCGTAT GTTGTGTGGA 450
ATTGTGAGCG GATAACAATT TCACACAGGA AACANCTATG ACCATGATNA 500
CNCCAAGCTA TTTAGCTGAC ACTANAGCAT ACTCAAGCTT GNATGCCTAC 550
AGNTCGACTC TAGAGGATCC ACCGGGTACC GAGCT 585

(2) INFORMATION FOR SEQ ID NO:8:

(i) SEQUENCE CHARACTERISTICS:
(A) LENGTH: 693
(B) TYPE: cDNA
(C) STRANDEDNESS: Single
(D) TOPOLOGY: Linear

(ix) FEATURES
(D) OTHER INFORMATION: Pyruvate decarboxylase

(xi) SEQUENCE DESCRIPTION: SEQ ID:8:
CATCTTTTCA CTCGAAGTCT CAATCTTTCA TCACAAACAT TCCCATTTGA 050
TCACAAAAAA GTTTCAACCT TTAAACCTCC ATGGACACCA AGATTGGCTC 100
CATCGACGTC TGCAAAACCG AGAACCACGA CGTCGGTTGT TTACCAAACA 150
GCGCCACCTC CACCGTTCAA AACTCAGTCC CTTCGACCTC CCTCAGCTCC 200
GCCGACGCCA CCCTCGGCCG CCACCTGGCA CGCCGCCTCG TTCAAATCGG 250
CGTCACCGAC GTCTTCACCG TCCCCGGCGA CTTCAACTTG ACCCTTCTCG 300
ACCACCTCAT CGCCGAGCCC GGCCTCACCA ACATTGGCTG CTGCAACGAG 350
CTCAACGCCG GGTACGCCGC CGACGGCTAC GCGCGGTCGC GTGGCGTCGG 400
CGCCGTTGCG TGGTGACTTT CACTGTTGGT GGACTGAGTG TGCTGAACGC 450
GATCGCCGGC GCGTTATAGT GAGAATTTGC CGGTGATTTG TATTGTTGGT 500

GGGCCCCAAC TTCTAATGAT TATGGGACTA ACCGGATTCT TCACCATACT 550
ATTGGGTTGC CGGACTTCAN TTCAAGAACT CCGGTGGTTT CAAGAACNTG 600
ACTTGCTTTT CAGGCTGTGG GTGAATAATT CTTGGAAGAA TGCACATGAA 650
TTTGCTTGAA TACNGCAATT TTCAATNGCN TTNGAAANAA AAC 693

(2) INFORMATION FOR SEQ ID NO:9:

(i) SEQUENCE CHARACTERISTICS:
(A) LENGTH: 693
(B) TYPE: cDNA
(C) STRANDEDNESS: Single
(D) TOPOLOGY: Linear

(ix) FEATURES
(D) OTHER INFORMATION: Chalcone reductase

(xi) SEQUENCE DESCRIPTION: SEQ ID:9:
CCCAAATCCC AGAAGTGGTT CTTGAATCCT CCAACGGCCG CAGAACCATG 050
CCTGTGCTTG GATTCGGCAC AGCATCCAAC AATTTACAAC CGGAGGTTTT 100
GATAGAAGCT GTTCTTGAGG CCATCAAGCT TGGTTACCGA CACTTCGACA 150
CTGCTTCCAT TTACGGCTCC GAGCAGACTC TAGGAGTAGC CATTGCCCAA 200
GCGCTCAAAC TCGGCCTCGT GGCTTCTCGT GACGAGCTCT TCATCACTTC 250
CAAGCTTTGG CCTAATGATG GTCACCCCAA CCTGGTTATT CCTGCTCTCA 300
AGAAAATCGC TTCAGAATCT TGAGTTGGAG TACCTTGATT TGTATCTGAT 350
ACACTGGCCC ATCAGTGCCA AGCCTGGGAA AGTTGAGTCA CGCACTAGAG 400
GGAGAAGGAC CAAATGCCGA TGGACTTCAA GGGTGTGTGG GCAGACATGG 450
AGGAAGCTCA GAGACTTGGC CTCACCAAAT CCATTGGGAA TCAGCAATTT 500
CTCTACCAAA AAGACTCAGA ATTTGCTCTC CTTTGGCTAC TATTCCTCCG 550
TCAGTCAATC AANTTTAANA TGANTCCATT TTGGCAACAG AAGAACCTCA 600

AAAACTTCTG CAAGGCCAGT GGTATAATTT GTGACTGGCT TCTCCCCATT 650
GGGTGCCATN NGAACCANTT GGGGGCACCA ATCATGTTCT CNA 693

(2) INFORMATION FOR SEQ ID NO:10:

(i) SEQUENCE CHARACTERISTICS:
(A) LENGTH: 763
(B) TYPE: cDNA
(C) STRANDEDNESS: Single
(D) TOPOLOGY: Linear

(ix) FEATURES
(D) OTHER INFORMATION: Protein kinase

(xi) SEQUENCE DESCRIPTION: SEQ ID:10:
GCANANCGTG TTGTGGGAAC TGGGTCATTT GGAATTGTAT TCCANGCGAA 050
ATGCTTGGAA ACTGGTGAGA CTGTGGCCAT AAAGAAGGTT TTACAGGACA 100
GAAGGTATAA GAACAGGGAA CTTCAATTGA TGCGCGTAAT GGATCATCCA 150
AATGTGATTT GTTTGAAGCA TTGTTTCTTC TCTACAACAA GCAAAAATGA 200
GCTTTTTCTC AATTTGGTTA TGGAATATGT TCCGGAAACT ATGTATCGGG 250
TTATAAAGCA TTACAGCAAT GCAAACCAGA AAATGCCCCT TGTCTATGTC 300
AAACTTTACA TGTNCCACAT TTTCAGAGGG CTGGCTTACA TACACACCGT 350
TCCTGGAGTT TGCCATANAN ATTTGAANCC TCCAAATTTA TTGGTTGATC 400
CTCTTATTCA CCANGTCAAG CTTTGTTGAT TTTGGAAGTG CCAAAATGCN 450
GGTGAAAGGN GAAACAAACA TANCATACCT ATGTTTCACG TTTCTATCNG 500
GCTCCNCGAA ACTAATTTTT TGGTGCCNCC NGATTATACC ACTTCCCATT 550
GATATCTGGT CNGCTGGCTG TGTCCTAANC AAAACTTCCT TTTGGGCCCC 600
CCTTTGTTTC CCTGGAAAAA AATGCCATNG AACCACCTGT TAAAAATCNT 650
TCCNGGTTCN GGGGAACACC NCNCCNTTCA AAAAATCCCC NTTTTGAATC 700

CCCANTTNTA CCAAATTCCC GGTTTCCNCC GAAAAAANCC CNCCCTTTGG 750
NNNAAGGTTT TCC 763

(2) INFORMATION FOR SEQ ID NO:11:

(i) SEQUENCE CHARACTERISTICS:
(A) LENGTH: 772
(B) TYPE: cDNA
(C) STRANDEDNESS: Single
(D) TOPOLOGY: Linear

(ix) FEATURES
(D) OTHER INFORMATION: Auxin-related gene

(xi) SEQUENCE DESCRIPTION: SEQ ID:11:
GGTGAAACTT TACTTTTGCA ATACACCGTC TAACAATGGC TGCAGCTCCA 050
AGTGAGTCCA TACCCTCTGT AAATAAGGCC TGGGTCTATT CAGAGTATGG 100
AAAAACTTCT GATGTTCTCA AGTTTGATCC AAGTGTGGCT GTTCCTGAAA 150
TTAAAGAGGA TCAGGTGCTG ATCAAGGTTG TTGCTGCTTC TCTTAACCCA 200
GTTGATTTTA AGAGGGCTCT TGGTTACTTC AAGGACACTG ACTCTCCCCT 250
ACCTACAATT CCAGGGTATG ATGTANCTGG TGTGGTGGTA AAGGTAGGAA 300
GCCAAGTAAC CAAGTTTAAG GTGGGGGATG AAGTGTATGG GGATCTCAAT 350
GAAGACAGCA TTGGTGAACC CAACAAGGTT TGGGTCTTTG GCANANTACA 400
CTGCTGCAGA TGAAAGANTA TTGGCTCACA AACCCAAAAA CCTGAGCTTT 450
ATTGAAGCTG CTANCCTTCC CTTGGCTATT GAAACTGCCC NTGAANGGCT 500
TGAAAGAACT GAACTTTCTG CTGGTAAATC CGTCCTTGTT TTGGGAAGCG 550
CTGGGGGTGT TGGAACACAN ATTATTCAGC TGCAAAGCAT GTTTTTGGTG 600
TTCCAAAGTA GCAGCTACTG CAAGCANTAA GAAACTGGAT TTGTTGAGAA 650
CNTTGGGNGC TGATTTGGCT ATCGATTACA CCAAGGAGAA NTTNGAGGAC [?]00

CTGCCAGAGA AATTTGATGT AGTGTATGAT GCAGTTGGGG AGACAGATAA 750
GGCTGTGAAG GCGGTGAAAG AAGGCGGGAA GGTTGTAACA ATAGTAGGTC 800
CAGCAACGCC ACCGGCTATC CTTTTTGTGC TTACCTCTAA AGGGTCTGTG 850
TTGGAGAAAC TGAAGCCTTA CTTGGAGAGT GGGAAGGTGA AGCCAGTTCT 900
TGATCCCACA AGTCCATATC CCTTTACTAA AGTTGTTGAA GCATTTGGTT 950
ACCTTGAGAG TTCCAGAGCT ACCGGAAAGG TGGTTGTGTA TCCCATCCCA 1000
TGAGGTTGAG AGTGTATGTG TGAATGATCT ATGAGACTAT GATTGTGTAG 1050
AGTCCATTTC CTTCCTCTTG TATGTGTGTA GCAGTATATT TTAATCTTGA 1100
AGCCTTGTAA TAATGAATAA GATTGAGTCC TTAATAAATT GTCATTACAT 1150
G 1151

(2) INFORMATION FOR SEQ ID NO:12:

(i) SEQUENCE CHARACTERISTICS:
(A) LENGTH: 1167
(B) TYPE: cDNA
(C) STRANDEDNESS: Single
(D) TOPOLOGY: Linear

(ix) FEATURES
(D) OTHER INFORMATION: Sucrose transporter

(xi) SEQUENCE DESCRIPTION: SEQ ID:12:
CCATTGGCGA TCACGTACAG TGTTCCATAT GCCTTGATTT CTTCTCGTAT 050
CGAGTCTTTG GGACTTGGCC AAGGCTTATC AATGGGTGTA CTGAATCTGG 100
CAATCGTAGT ACCACAGGTG CTGGTATCCC TGGGAAGTGG ACCATGGGAT 150
CAGCTATTGG TGGTGGAAAC TCTCCAGGGT TTGCGGTTGC AGGAGTTGGA 200
GCCTTAGCAA GTGGGCTGGT GGCCAATCTT GGCTATTCCA CGTTCTATTC 250
CACAGAAGCC TANATCTTTC ACATGAGGTA TTTTGTTGTA TCTACTTTTT 300

ACCCAACTTT GTCACAGAAA TACAAAACCT CCATAGATAG TGAGAATTTG 350
TAAATATCTT TTGTTACGTG TTAGCTATTT CTCAATACAC TCATTTACCA 400
GAGGTTTCTT TAGTTCTGGA AATTTCTCTC TTTCCCTTTT TGTCGTTTTA 450
GATGCTTTAA TAAAGAAAGG CCTGGCAGCG ATTATATCAA AGTTGANCTG 500
AATATCTGTG TTGAAGTGCT TCCGTTCAAC AATTTATAGT TCTCAATTTC 550
TACAATATTT TAAATCAGAA CTGTCACCTG GTGGACTCTT ATGGAATCCA 600
TATGTTGGAA CCATAATCTC AATTAGGCAT CGTGCCTCAA TTCCACAATG 650
GTGTTTTCAG AAGTGTGATG AAACAAGTTA GTCAAGAAAG TGATGGTGTT 700
TTCACAAATG CTGGCTACGC AACGATATTG ATGTGGGTAC GCAAATTGAT 750
TGATGTAGTA GCCATCACTA AGTTCCTGGT TAGACAAGTT ATCTACAATT 800
AGTGGANAAT TTCTTGAATG AAAATCAGTC CCATCTGGTG GATTGTGGCA 850
AATTGCTACG GAAAAGTAGG TGAAGCCTCA GCTGTAGGAT TTGGAAATTA 900
CTTGAAGAGT AGTTCCCTAC CAACCAGGAT ATGTTTCTGC TTTTCGAGAA 950
TTTGTCCTCC TGAAAATATC GTTTTTTCTT TTGGCAAAGT TGATTTTGAC 1000
TTAGTGGTTT AATCATGAGG TATTGGAATC TCATGCGTTT TGTGCATGTA 1050
TTTGTANTAT GAATGTGGTG AAATGTGCTT GGTGGCCAAC AGTGAATATA 1100
TGAAATGTAC TGATTGAAAC CTTGATGGAN ACATCCCTTT TAATTGCTGT 1150
TTTGGAAGCT TGGGTCC 1167

(2) INFORMATION FOR SEQ ID NO:13:

(i) SEQUENCE CHARACTERISTICS:
(A) LENGTH: 476
(B) TYPE: cDNA
(C) STRANDEDNESS: Single
(D) TOPOLOGY: Linear

(ix) FEATURES
(D) OTHER INFORMATION: meristem pattern gene

(xi) SEQUENCE DESCRIPTION: SEQ ID:13:
CCTCANNAAT CTCTATATTT TTTGGGGGCG TGGGTGGTCT AANAATATGT 050
TCTTGGCTTC AAAACCCTCA TCAGATGGAG AGCACCGACT CGTCTTCCGG 100
CTCGCAGGCG CCGCCGCAGC CAAACCTACC TCCGGGATTC CGCTTCCACC 150
CCACCGATGA GGAGCTAGTC GTTCATTACC TCAAGAAAAA GGCCTCCTCG 200
GCTCCCCTCC CCATTGTCAT CATCGNCGAA GTCGACCTCT ACAAATTTGA 250
TCCATGGNAG CTCCCAGAAA AGGCGACGTT CGGAGAGCAA GAGTGGTACT 300
TTTTCAGTCC TAGAGACCGG AAAGTACCCN AACGGAGCAC GGNCTAATAG 350
AGNAGGGACT TCAGGNTTTT GGTAGGGGAA CCGTANTGAA AAGCCCTTTT 400
GGGTTGNACT ATTANGAGGN NGGGGGNTCT CCCAAANTTG NGGTNAAAAN 450
GNANTTNTTT NTTTNANGGG ACNNCC 476

(2) INFORMATION FOR SEQ ID NO:14:

(i) SEQUENCE CHARACTERISTICS:
(A) LENGTH: 497
(B) TYPE: cDNA
(C) STRANDEDNESS: Single
(D) TOPOLOGY: Linear

(ix) FEATURES
(D) OTHER INFORMATION: transcribed sequence, T45086

(xi) SEQUENCE DESCRIPTION: SEQ ID:14:
TNAATTAANG GCAGCCNATT CGGTGAATTT CCTTCATTCG ATCCTGCAAA 050
CATGCCTTAT GGNAACGCTT GAAGTCCTTC TGGTTGGGGN CAAAGACCTT 100
GNAGACCATG ATTTTTTCGG TAAAATGGAT CCCTATGTCC TTTTATCATT 150
AAGGACCCAA GAGAAGAAGA GCACTGTGGC ATCAGGACAA GGATCTGCAC 200

CAGNANTGGN AATGAAACTT TTCAATTCAC AGTCTCATCA GATGATGTTA 250
CCGAACTCAG CTTAAAAATC TATGACAAAG ATACCTTCAC CCCAGATGAA 300
TTTCTTGGAG GAAGCAACCA TTCCTTTAGN AAACAGTGTT CATGGGAAGG 350
AAGCACTGAA CCGACTAAAT ACAATGTCGT CAATGAGAAT AATGAATATC 400
ATGGAGGATA TTACAGTTGG ACTCACTTTC ACCCGTGAAG CGAACCGGCT 450
CTCGTGCGGG NGGNTNTGAT GAAGAAAGAA CAA 483

(2) INFORMATION FOR SEQ ID NO:15:

(i) SEQUENCE CHARACTERISTICS:
(A) LENGTH: 488
(B) TYPE: cDNA
(C) STRANDEDNESS: Single
(D) TOPOLOGY: Linear

(ix) FEATURES
(D) OTHER INFORMATION: transcribed sequence L36159

(xi) SEQUENCE DESCRIPTION: SEQ ID:15:
AGGATATGTT GATTAGAACT CATGTAACCT CATATTACAC ATCTTAATAT 050
CTCCAATTAC ATGAACGTAA AATAAAACCC CTAACTTCCA CAAAGCATCA 100
ATCANACACG GGGNACGTCC GCGAATGCTA AGCAACTTGA CATCATCGAT 150
CACCGGACCA CACAGAGAGC CGGAGTGATC GCTCGTCATG GTGTACATTG 200
TGCTCAGAAA CATGACACGC GTGCGCGGCG NACACGGNGG TGNAAGAAGA 250
GCCTGGCCTT CTTGNAACCC TCCTTTGCCT TTGGACTCAT AAGGAACCTT 300
CACAGTCTCC TTGCCGGCAA ATGCCTCGAT AAAGAGGGAG CCTTCGCAGT 350
CGTTGGTTCC CGTCGNCGAC AGAGAATNTN AGGGCGTAGC GCCTNNCGGG 400
NTTGGTGAAG ACCACTTGAG CCAATGNGCT CTCTTTTCCC GGCAACGAGC 450
TCGNTNGGTN TTAGGCCTCC NGGANGGGAA GTGTGGNG 488

(2) INFORMATION FOR SEQ ID NO:16:

(i) SEQUENCE CHARACTERISTICS:
(A) LENGTH: 460
(B) TYPE: cDNA
(C) STRANDEDNESS: Single
(D) TOPOLOGY: Linear

(ix) FEATURES
(D) OTHER INFORMATION: transcribed sequence, T45902

(xi) SEQUENCE DESCRIPTION: SEQ ID:16:
GTTTGTCCTC GGTTCCTAAA GAGAGAGACA CCCAGAATTT GNTTCAGAAA 050
TCGGAGATTA AGTTCCTGAA CCAAGTTCAA GGCCCTGAGA GCGTCGCCTT 100
TGATCCACAA GGACGTGGAC CATATNCTGG TGTTGCGGAT GGGAGGAGTC 150
TTGTTCTGGA ATGGGCAGGC CTGGACTGAT TTTGGCTATN CATCGCCNCA 200
CAGGTCAAGA TATATGTGNA TCCCANAACC ATCAGCTATG ANTTACTTGG 250
CAAATGAGCA CATCTNTNGN AGGGGCCNTG GGGCTCCCCC TTTTGGNAAA 300
GAAAACAGGA GATTTGGNGC AATTTGGGGG TTGAATACTT TTGGGCTTTN 350
TTNGAAAATG GGGCAAANGN TNNGGTTTNG GGAAATTTCC ACTTCNAACT 400
TAGGANAANG GGGNGCCATG NGGGTTTCTT ACCCTCTTGG NNTGGTGAGG 450
ANGGANAATT 460

(2) INFORMATION FOR SEQ ID NO:17:

(i) SEQUENCE CHARACTERISTICS:
(A) LENGTH: 480

(B) TYPE: cDNA
(C) STRANDEDNESS: Single
(D) TOPOLOGY: Linear

(xi) SEQUENCE DESCRIPTION: SEQ ID:17:
NTGGGTTCCA TGACACTTCC TAAAGAGCTT CCCACCATCA ATTTCTCCCT 050
CCAAGACTTG AAGCCTGGCT CAAGCTCCTG GACTTCCACC TGCAAACAAG 100
TCCGCAATGC ACTCGAAGAA TATGGTTGCT TTGTGGCATT GTNCCCACAA 150
GTCTCCCAAG AGCTCATGGA CAGTATCTTC GGNCAATCCA GGGATCTGTT 200
CGAGGTTCCC CTCGAGAACA AGGTCAAGAA CACCAGCGAG GAGCCTTACC 250
GTGGNTATAT CGGACCAAAC CCCCTCTTGC CACTCTATGA AGGCATTGGC 300
ATTGACAACG TCACATCCCA ACAAGAAACT CAGAAAGTTC AGGGACCTCA 350
TGTGGGCTAA TNGAAAGACC CAATTCTGTG AAAATCACAG ATCTTGTTNG 400
GCANGTNGCT CGGGGAGTTN GGAAAACACT GTGGAAANGA TGNTNTTNCG 450
NAAGTTACGG GNTACCTCTT GGGGANNTNA 480

(2) INFORMATION FOR SEQ ID NO:18:

(i) SEQUENCE CHARACTERISTICS:
(A) LENGTH: 673
(B) TYPE: cDNA
(C) STRANDEDNESS: Single
(D) TOPOLOGY: Linear

(xi) SEQUENCE DESCRIPTION: SEQ ID:18:
GATTCGGGTA CANTTACAGT ACCAGATATC AATATCAATA CTAGATAACA 050
GTATATNGCA CGTCTTCTTC TTCTTCTTCT TCTTCTTCTT CTTTTTTGGT 100
GGAAGCTCGT CTTCTTCTTC TTCTAGCTAG CTTTCTTCAG CTTTTTTTAT 150
TTGTTATTCT TCATCTTCTA CCCTAATATA CTCTTTGATA CATAAAAGTC 200

CAGCACTTTT CAAACAATAG CAACTCAGTA GTCTTTACCC TCAGTAGTGA 250
TTAAAAACTA CTGCGTCGTC ACTCCACAAG AGCTTGTATT ACCACNTAGA 300
TGGCCTCATT GCGCTCTCTC GCATTCCAGG TGAATCACTT CGAGCTGCAA 350
CTTATAACGC CGGCAAAGNC AACACCGCTC GAAATGAAGC TGTTGGTCGA 400
ATATCGACGG ACCAGCAATG CCTCAGGTCT CATGTTCCCC ATTCATCATG 450
TCTTACAAGA ACAATCAATC AATACTGTCG GAAACCAAAC GACCCGNNGG 500
AGGTGGATTA GGGGATGCGC TGAGCAAGGG ACTGCAGTTT TACTACCCCT 550
TGGGTGGTNG GTTCANGGNG GGGCCTAACA AAAGGNTATG GNGGACTGAA 600
CCGNGAAGGA ACTTGGTCGN TGGGGGAACG CCGAGGCAAA NCGAGGACTC 650
GGGNTGAACC CANCGCCNGG CCA 673

(2) INFORMATION FOR SEQ ID NO:19:

(i) SEQUENCE CHARACTERISTICS:
(A) LENGTH: 749
(B) TYPE: cDNA
(C) STRANDEDNESS: Single
(D) TOPOLOGY: Linear

(xi) SEQUENCE DESCRIPTION: SEQ ID:19:
AAATTGAGGT CAGTATAAAT TCCAAACACA CCATCAAACC ATCAACTTCC 050
TCTACACCAC TTCAGCCTTA CAAGCTTACC CTCCTGGACC AGCTCACTCC 100
TCCGGCGTAT GTCCCCATCG TATTCTTCTA CCCCATTACT GACCATGTCT 150
TCAATCTTCC TCAAACCCTA GCTGACTTAA GACAAGCCCT TTCGGAGACT 200
CTCACTTTGT ACTATCCACT CTCTGGAAGG GTCAAAAACA ACCTATACAT 250
CGATGATTTT GAAGAAGGTG TCCCATACCT TGAGGCTCGA GTGAATTGTG 300
ACATGACTGA TTTTCTAAGG CTTCGGAAAA TCGAGTGCCT TAATGAGTTT 350
GTTCCAATAA AACCATTTAG TATGGAAGCA ATATCTGATG AGCGTTACCC 400
CTTGCTTGGA GTTCAAGTCA ACGTTTTCGA TTCTGGAATA GCAATCGGTG 450

TCTCCCGTCT CTCACAAGCT CCATCGATGG AGGAACGGCA GAATGTTTTC 500
TCAAGTCCTG GGGTGCTGTT TTTCCGAAGG TTGTCCGTGA AAATATCATA 550
CATCCCTAAT CTCTCTTGAA AGCCAGCATT GCTTTTCCCC ACCGAAAANA 600
TGACTTGCCT GAAAAGTTAT GCCGATCAGA TGGAAGGGTT ATGGTTTGCC 650
CGGAAAAAAA TTGCTACAAG GAAATTTGTA TTTGGTGTNA AAACCATATC 700
TCCATTCCAG AAGAAACGAA AACGANTCCG TGCCCAAGCC ATCACAATT 749

(2) INFORMATION FOR SEQ ID NO:20:

(i) SEQUENCE CHARACTERISTICS:
(A) LENGTH: 218
(B) TYPE: cDNA
(C) STRANDEDNESS: Single
(D) TOPOLOGY: Linear

(xi) SEQUENCE DESCRIPTION: SEQ ID:20:
TCGGGCAGAG AAATCTTTGA GATTGGCAGA CTCGAGAGCA TCCAGACTTC 050
GAGAAAGAGT AGAGGAGCTT ACCTGTCAAC TGGAAGAATT TGAAAATCGG 100
GAGGACTTAA GGAGAGGCCT GGGTGGACCT AGATATGTAT GTTGGCCCTG 150
GCAGTGGCTT GGGCTGGACT TTGTAGGGTT CAGTCGCTCT GATACAGAAC 200
AACAGAATAG TTCAAACG 218

(2) INFORMATION FOR SEQ ID NO:21:

(i) SEQUENCE CHARACTERISTICS:
(A) LENGTH: 437
(B) TYPE: cDNA
(C) STRANDEDNESS: Single

(D) TOPOLOGY: Linear

(xi) SEQUENCE DESCRIPTION: SEQ ID:21:
CGCTGCTTGT CTCGCTGCCT ACTATCACTA CTACCATGGG TTGGTCCCCT 050
TTCCTTCAGA ATCGGACATG TTTTGGGACG TTCAGATTCC ATCTATGCCG 100
CTGTTGAAGT ACGATGAGGT ACCCAGCTTC TTGTACCCTA CTAGTCCTTA 150
CCCGTTTTTG AGGAGGGCCA TTTTGGGACA ATACGGGAAC TTGGAGAAGC 200
CCTTCTGTAT ATTGATGGAC ACTTTCCAAG AACTCGAGAG CGAGATCATC 250
GAGTACATGG TTCGTTTGGT GCCCCATCAA NGTTGTTGGT TCCCCCTTCT 300
TTCAAAGAAC CCCAAAAGCC CAAAANCGCT NTTCCCCCGG GGGATTTCCA 350
TNAGGGCCGA CGNANTTCAN CCANCCGGTT NGTTTCGGAA ACNAAAACNN 400
AACANNTTTC GNGGNTTTTT NACACCCANG NTNNCGG 437

(2) INFORMATION FOR SEQ ID NO:22:

(i) SEQUENCE CHARACTERISTICS:
(A) LENGTH: 232
(B) TYPE: cDNA
(C) STRANDEDNESS: Single
(D) TOPOLOGY: Linear

(xi) SEQUENCE DESCRIPTION: SEQ ID:22:

AAGAAAGGAG TCTCGTCAAT AAAGGATTTG TGAGAATCAA ATAACGTTCT 050
CTGTTTATTA ATTTGTAACA GTAGTTTGAT CGAGTCTGTG AGTAAGTGAT 100
CGAGTAAGAG ATGTACTCTA CTGTGTGTGT GTCAATCATG TTCGTGTTCT 150
TTGGTAGCCA TGTAATGTTC TCCATCTGGT CATTATCTGT GGCCTTGTGA 200
TCATGTTTAA TCAATGAAAC TACTATTAGT AT 232

(2) INFORMATION FOR SEQ ID NO:23:

(i) SEQUENCE CHARACTERISTICS:
(A) LENGTH: 469
(B) TYPE: cDNA
(C) STRANDEDNESS: Single
(D) TOPOLOGY: Linear

(xi) SEQUENCE DESCRIPTION: SEQ ID:23:
GATCGGTCCG ATGACCGGAA AAGTCATGAT TTTGAGGTGA GGAGCGANTT 050
GGGTTTCGCC NGAAATGTNC AAAGCCCTGT GCTTTCGGAG CATGTGGTTG 100
AGAATTTGGN GAAAGGCAAA GTGGGTGTCC AAGAAATTGG NGAANTTGGN 150
AGCTTTGATA AGGATTTGGG ATAANTTCTN GTTTGATTCC CGCCNGAGAA 200
AGCTCGNTCT TCTTTTGAAA TTTGACAANG AGGAGGGGTT CANCNCNAGT 250
CCAACAANNG AATCAAGGGA GGANANACTC ANCTTNAGAC TCANCGTTCG 300
CNCAGANGNA GNAANNTAAA AACTGNGGCG AAAACCGNCT NNCGAGGTGA 350
TAATTAANNT CCACCTTCTT TNTTNCACGG TCCCCCCGCT TNTTTTTNNA 400
GCTTTTTCTC CNTCAANGCN AATTCCCGTT NGNTNTTCTT NTTNTGCCNA 450
NNCTAATNCN CTTNATTCC 469

(2) INFORMATION FOR SEQ ID NO:24:

(i) SEQUENCE CHARACTERISTICS:
(A) LENGTH: 178
(B) TYPE: cDNA
(C) STRANDEDNESS: Single
(D) TOPOLOGY: Linear

(xi) SEQUENCE DESCRIPTION: SEQ ID:24:
AACCAGATAT NAAGCGATTT TCGATATTCA ATAACATTCT TCTTTAACTG 050
TTCAGGTGCG TCAGGAGCCC AACGCTCAGG GTAATCGGCG AAAGTGAATN 100
TTGGNTNGAC ATTAGNAACC AGCCAGACCA ATAGCCGTTG GAACAGCTGA 150
CGTTCGGCGC GCCCAACCGG TGGNGCAA 178

(2) INFORMATION FOR SEQ ID NO:25:

(i) SEQUENCE CHARACTERISTICS:
(A) LENGTH: 244
(B) TYPE: cDNA
(C) STRANDEDNESS: Single
(D) TOPOLOGY: Linear

(xi) SEQUENCE DESCRIPTION: SEQ ID:25:
TTCAAGTTAA CCTCTCAAAC CCGACACAGA GAGCATAAAT GGGTTCCGAA 050
TCATTGGTTC ATGTTTTCTT GGTTTCCTTC ATCGGCCAAG GCCATGTGAA 100
CCCACTCCTC CGNCTCGGNA AGCGCCTCGC TGNCAAAGGT CTCCTCGTCA 150
CCTTCTGCAC CGTCGAATGC GTCGGTAAGG AAATGCGNAA GTCCAACGGC 200
ATCACCGACG AGCCCAAACC AGTTGGAGAT GGATTCATCC GCTT 244

(2) INFORMATION FOR SEQ ID NO:26:

(i) SEQUENCE CHARACTERISTICS:
(A) LENGTH: 685
(B) TYPE: cDNA
(C) STRANDEDNESS: Single
(D) TOPOLOGY: Linear

(xi) SEQUENCE DESCRIPTION: SEQ ID:26:
CCAATTCGGT CGCCGTAAAA CATGGTTAAT CAAACGGTGA ACGGAAGCCA 050
ATCAAGTAGC GGAACCCAAA AGCTCAATGC TTCAAGCAAC ACCAAGAGGG 100
ATTTTGAGGC TGTGAGTGAG TCCATGCACT CTGCAATTTC AATGAGTAAA 150
ACAGAAGTCT TGGATTCTGT GCTGAGTGAT TTCTCTGAGG GATATTTTAG 200
CCTTTGCTAT GAGAATCGTC GAAAATTGCT TGTGCAACTT GCCAAAGAGT 250
ATGATCTTAA CAGGACNCAG GTTCGCGATT TGATAAAGCA GTATTTGGGA 300
CTTGAGCTTC CTGGAACTGG AAGTGACAAT GCTGACTCAG AAAGAGGAGG 350
CATCTCTTTC TGCTTTCTAC CGCATTGANA GGAACTTGAA GACNTGCTCT 400
CNAGCCCATG TATGAANTGC TATTTGAGCG GCTTAATACG CNTCCCGGAG 450
GGTTGAAGTT CTTGTCTATT CTTTCGAGCT GATATCTTTA TCCATTCTCG 500
CANAAAAATA ATCTGGCGTC TTTGCNAACA TTGGATTCCC CATTCAAAGG 550
AGAAACTTAN TNCGTTGGTT AATCCCCCTG CCTTANNAGC TCCNCCCCCA 600
TCNCTCGGAT GATTCTTCCT CCCTTTGCTG GGAAAAAATT GTNGCTTACT 650
AAGGCCGTGC TTCCCATCCA NCTATTCTTC TNGAT 685

(2) INFORMATION FOR SEQ ID NO:27:

(i) SEQUENCE CHARACTERISTICS:
(A) LENGTH: 668
(B) TYPE: cDNA
(C) STRANDEDNESS: Single
(D) TOPOLOGY: Linear

(xi) SEQUENCE DESCRIPTION: SEQ ID:27:
AAGTTCACCC AAGTATAAAG TCTATCTTAT TATATATACG TTTTCTACAA 050
TGTTTGACTA TTACTGATAT TANAANATCA GCTTAAGGAG CAACAAACAT 100
ATTATTACAT TATAATGACA ACAGTACATT GATAATCACT TTCCACTATA 150

GAAAACAACA AAATTAAAAG TGTGGACACA TCCGTTATTA CATTGCTACC 200
CGGCTATTCT GTTGTATTTT GAGGTTCCTT CAGTGGCTCA ACGTAACGGG 250
AAAGTACATT AAAANTATGG ATATGCCCTG TNCTGAAATA TGACTGAAAA 300
TAATCTTCAA TGTTGCCCAA TCTGTAAACA TAGTTCACCA TGATACCTCC 350
ACTTTGATNA AGGCCTTTAT CTGATCGATC AGCCATCCNA TTAATTCTCT 400
CAACCATTGC TCCATTCTGT NAGTTGAAAA TTTGCAACAG AATCCANAAC 450
TTTGCCTCTC TTTTTCTCTT GCAAAAANGT ANCTGGCACA CAATCCCATT 500
AAAAAGGGGT TTTTAGAACT GAAAACCAAT TTATCANAAC TTTGTTCCCT 550
CCCGGGTTTG CTGAANTTCC GTAAATTGAN CATCCCTCCA TGCCGTTTTT 600
TCCCCNTGGG TGAATTCAAA AAACCTNCTC NAAAANTNTT TCTAAAACNG 650
GCGCGGGGCC ATNCATTT 668

(2) INFORMATION FOR SEQ ID NO:28:

(i) SEQUENCE CHARACTERISTICS:
(A) LENGTH: 522
(B) TYPE: cDNA
(C) STRANDEDNESS: Single
(D) TOPOLOGY: Linear

(ix) FEATURES
(D) OTHER INFORMATION: O-methyl transferase

(xi) SEQUENCE DESCRIPTION: SEQ ID:28:
NTNNGGGGGT TGGGGNTCTN GAAGGCAAAA GATTCGGTCA GGACAAGGTC 50
CTCGTCGAGA GCTGGTATCA TTTGANGGAT GCAGTTCTTG ATGGTGGGAT 100
TCCATTTAAC AAGGNCTATG GCATGACTGC ATTTGATTAC CATGGNAACT 150
GACCCTAGCA TTCAACAAGG TCTTCAACAA GGGAATGGCT GACCACTCCA 200
CCATTACCAT GCANGTAAAA TCCTTGTAGT ACTTACAAAG GCTTCGAGGG 250

CCTCAAATCC ATCGTTGTAT GTCGGTGGCG GNACCNGAGC TGTGGNGGAA 300
CATNATCGCT TCCCNAGTTN CCCTTCGCAT CAAGGGTCAT CANCCTTTCG 350
ACTTGCCCTC AATCTTANTC GAANGCATTC CTCCNTCAAT TATCCTNNNT 400
GTTTCCANCC ANGTTGGGAT GNGGGGANAA TCTTCTGGCN ANNTCTTACC 450
CAATTNNGGN ANNCTTCCAT TCTTTCCCAT TTNAGTTCNT NTTTTNCTCA 500
ACCTAACTTG NCGNTCCNTC GN 522

(2) INFORMATION FOR SEQ ID NO:29:

(i) SEQUENCE CHARACTERISTICS:
(A) LENGTH: 445
(B) TYPE: cDNA
(C) STRANDEDNESS: Single
(D) TOPOLOGY: Linear

(ix) FEATURES
(D) OTHER INFORMATION: Acyl carrier protein

(xi) SEQUENCE DESCRIPTION: SEQ ID:29:
ATGGCCACCA CCACAGGAGC TGCTTCTTCG ATCTCACTCC GCTCTCGCCT 50
TCACCAGAAT CTTGCATTGT CCAGGGTCAA TGGTCTTAAG CCAGTTTCAC 100
TGTCTGGTAA TGGAAGAAGT TCTCTTTCTT TCGGGTTACA GCAGCGTTCA 150
GTACGGCTTC AGATTTGCTG CGCGGCCAAA CCAGAGACAG TGGACAAGGT 200
GTGCCAGATA GTTAGAAAGC AACTTGCATT ACCAGATGAC TCAGCAGTTT 250
CTGGAGAGTC AAAATTTTCT GCACTTGGAG CTGATTCTCT TGATACGGNN 300
GGAGATTGTG ATGGGACTTG AGGAGGAATT GGGTATTAGT GTGGNNGAGG 350
AGAGTGCTCA GAGCATTGAA CTTNTNCAAG NTGCTGGGGT CTTTTCNANA 400
AGNNCNATNG NAAGACCAGG NTTIGGAGGA GGANTNANAA ACAAG 445

(2) INFORMATION FOR SEQ ID NO:30:

(i) SEQUENCE CHARACTERISTICS:
(A) LENGTH: 562
(B) TYPE: cDNA
(C) STRANDEDNESS: Linear
(D) TOPOLOGY: Single

(ix) FEATURES
(D) OTHER INFORMATION: Elongation factor 2

(xi) SEQUENCE DESCRIPTION: SEQ ID:30:
GGATCATCCC TTGGNCCAAT ACGACCATCA TCAATGGNCT CAGGAAGACC 50
TTCCTCCAAC GGGNGTGCTT CCATGTACAG ACGGTTGTGC TTGTTGGGAG 100
ACTTGCTCAT CACAGTACGG NAGGNCTTCT CAAGGACTGT CTCACGGNAG 150
GACACAACAG GATCAGATTT TACAATTTCC GCTCCACCCA TAAAATCATC 200
TTGNAGATCC NTCANGNAGA TCTCAAGGTG AAGTTCACCA GCTCCAGCAA 250
TGATGTGCTC TCCAGACTCC TCAATGGTAC AGACACCCAT AGGGATCGGG 300
TCTTAGCCAG ACGTTTCAGC CCTTCAACAA GCTTGGGGAA GGATCAAGAA 350
GCANCCTTAC GNTTGAACAG CAACACGCAC AACAGGGTTG AGACAGGAGA 400
ACTTCATTGC ACGAATGGGG GGAGCATCTT GTTNCCCTCT CATTTGGTCA 450
AGGTAGCATT CTTGGGTGGA TTGAACTTNT TCCCAGACCA ACCAAGGGNA 500
CAAGTTTTTA CCACAGGGGA ACATCCTCAA CAGTTTCNTT GTTTCTTTTC 550
CCCATCCAGG TT 562

(2) INFORMATION FOR SEQ ID NO:31:

(i) SEQUENCE CHARACTERISTICS:
(A) LENGTH: 490

(B)	TYPE:	cDNA
(C)	STRANDEDNESS:	Single
(D)	TOPOLOGY:	Linear

(ix)	FEATURES
(D)	OTHER INFORMATION:	Auxin-induced mRNA

(xi)	SEQUENCE DESCRIPTION: SEQ ID:31:
ATCGACTGCA TTAAGTTGCT AGAAGTGGAG CTTGGTGACA AGCCTTTCTT 50
TGGCGGTGAG ACCCTCGGAT TTGTGGACGT GACGCTCGNT CCTTTCTATT 100
CCTGGTTCTC TGTGTATGAG AAATACGGCA ACTTCAGCAT TGCGCCAGAG 150
TGCCCAAAGT NCATGGCTTG GGTTAAGAGG TGTATGGAGA AGGAGAGTGT 200
GTCAAAGTCT CTTCCTGACC AGGACAAGGT CTGTGGCTTN GTTGCCGAGA 250
TGANGAAGAA GCTTGGAGTT GAGTAGATGT GATCAATGTC ATNTTGATCA 300
TGTCTTTGTT TTAGCCCCAA GATTCANCCT CGTTTTGGGT TGCTTGTATT 350
TTTCAATAAA ATTGGGGGAC TTGGACCAAG CCCTCCAATA GTAGGAAGCA 400
CTCTTTCNGT GCCTCTTGGT CCNGT1TTTC TTCNGNTAAN CCTNTNTGCA 450
GCTAAAATTC ACCGNATTNC TGNTTTCCTT NTATNGCCAA 490

(2)	INFORMATION FOR SEQ ID NO:32:

(i)	SEQUENCE CHARACTERISTICS:
(A)	LENGTH:	483
(B)	TYPE:	cDNA
(C)	STRANDEDNESS:	Single
(D)	TOPOLOGY:	Linear

(ix)	FEATURES
(D)	OTHER INFORMATION:	Cysteine (thiol) proteinase

(xi) SEQUENCE DESCRIPTION: SEQ ID:32:
GGATCTCCTC CTCCTCTCTC TCCTTCTCCT CCTCTCCTCC GCCGTCGCCT 50
CCACCGTAAC CGACGCCGGC GATCCTCTCA TACGACAAGT CGTACCGGGC 100
GCGGCCGAGG ATGACGAGCT CCTCCACGCG GAGCGTCACT TCTCGAACTT 150
CAAAGCCACG TTCGGAAAGA GCTACGCGAG CCAGGAGGAG CACGACTACA 200
GGTTCCGGCG TATTCAAGGN CAACTCCGCC GGGCGAAGAG GCACCAGGGG 250
CTTGGACCCC ACCGCCGTGC ACGGTGTCAA CGAAATCTCC GATCTCACTC 300
CCAAGGAGTT TCGNCGGGAA TTTCCTCGGG CTTAAGAAGG GGTCGGANTT 350
CGGGTTACCG GCCGACGGTT AAAAAAGGGG CCNGATNCCT NCCGGANGAA 400
TTANCTTCCC CACCCANTTT TGGNNTTGGG GNGAAAAAAG GNGCCCGNCN 450
AAGNCGGNGG AANGGNCAAG GGGGAAATNG GGT 483

(2) INFORMATION FOR SEQ ID NO:33:

(i) SEQUENCE CHARACTERISTICS:
(A) LENGTH: 520
(B) TYPE: cDNA
(C) STRANDEDNESS: Single
(D) TOPOLOGY: Linear

(ix) FEATURES
(D) OTHER INFORMATION: Cellulase (endo-(1,4)beta-n-glucanase

(xi) SEQUENCE DESCRIPTION: SEQ ID:33:
ACGGTGGGGG GACAGACTAC CTCCTGAAGG CCACGGGGGT TCCTGGCGTC 50
GTCTTCGTCC AAGTCGGCGA CCCATACTCC GATCACAACT GCTGGGAGGA 100
GGCCGGAAGT ACATGGTACA CACGCCGCAC GGTGTACAAA ATCGACCACA 150
ACAACCCGGG ATCCGACGTG GNAGGTGTAA ACCGCAGTTC GTGCTCGCCG 200

TCGCCTCTAT CGTTTTCAGG TCACGTGACC CCGCTTACTC GNAGNACTGC 250
TTCTCAATCG GAGCCGTTAA GGTTT1CGAG TTCGCTGATA CCCACCGTGG 300
TGTGTTCAGA TCCAGCCTCA AAAACGCCGT TGTGCCCCTT TTTTACTGTG 350
NAANGTCAAA CGGNTTTCCA GGGATNAATT TACTNTTNGG GGAGGNAGCG 400
TTTGTTTGGN ACAAAGGTGG TCTATTNGGC NGGAGTACAA GTAGTATTNT 450
CATTGTGNTN AATCGGANGN CTATTTTGGG GGAGNTTTNA GGNTNCCMNT 500
TAANGAANTT TGNNTGGGCT 520

(2) INFORMATION FOR SEQ ID NO:34:

(i) SEQUENCE CHARACTERISTICS:
(A) LENGTH: 695
(B) TYPE: cDNA
(C) STRANDEDNESS: Single
(D) TOPOLOGY: Linear

(ix) FEATURES
(D) OTHER INFORMATION: Pyruvate decarboxylase

(xi) SEQUENCE DESCRIPTION: SEQ ID:34:
GGCATCTTTT CACTCGAAGT CTCAATCTTT CATCACAAAC ATTCCCATTT 50
GATCACAAAA AAGTTTCAAC CTTTAAACCT CCATGGACAC CAAGATTGGC 100
TCCATCGACG TCTGCAAAAC CGAGAACCAC GACGTCGGTT GTTTACCAAA 150
CAGCGCCACC TCCACCGTTC AAAACTCAGT CCCTTCGACC TCCCTCAGCT 200
CCGCCGACGC CACCCTCGGC CGCCACCTGG CACGCCGCCT CGTTCAAATC 250
GGCGTCACCG ACGTCTTCAC CGTCCCCGGC GACTTCAACT TGACCCTTCT 300
CGACCACCTC ATCGCCGAGC CCGGCCTCAC CAACATTGGC TGCTGCAACG 350
AGCTCAACGC CGGGTACGCC GCCGACGGCT ACGCGCGGTC GCGTGGCGTC 400
GGCGCCGTTG CGTGGTGACT TTCACTGTTG GTGGACTGAG TGTGCTGAAC 450

GCGATCGCCG GCGCGTTATA GTGAGAATTT GCCGGTGATT TGTATTGTTG 500
GTGGGCCCCA ACTTCTAATG ATTATGGGAC TAACCGGATT CTTCACCATA 550
CTATTGGGTT GCCGGACTTC ANTTCAAGAA CTCCGGTGGT TTCAAGAACN 600
TGACTTGCTT TTCAGGCTGT GGGTGAATAA TTCTTGGAAG AATGCACATG 650
AATTTGCTTG AATACNGCAA TTTTCAATNG CNTTNGAAAN AAAAC 695

(2) INFORMATION FOR SEQ ID NO:35:

(i) SEQUENCE CHARACTERISTICS:
(A) LENGTH: 695
(B) TYPE: cDNA
(C) STRANDEDNESS: Single
(D) TOPOLOGY: Linear

(ix) FEATURES
(D) OTHER INFORMATION: Chalcone reductase

(xi) SEQUENCE DESCRIPTION: SEQ ID:35:
GGCCCAAATC CCAGAAGTGG TTCTTGAATC CTCCAACGGC CGCAGAACCA 50
TGCCTGTGCT TGGATTCGGC ACAGCATCCA ACAATTTACA ACCGGAGGTT 100
TTGATAGAAG CTGTTCTTGA GGCCATCAAG CTTGGTTACC GACACTTCGA 150
CACTGCTTCC ATTTACGGCT CCGAGCAGAC TCTAGGAGTA GCCATTGCCC 200
AAGCGCTCAA ACTCGGCCTC GTGGCTTCTC GTGACGAGCT CTTCATCACT 250
TCCAAGCTTT GGCCTAATGA TGGTCACCCC AACCTGGTTA TTCCTGCTCT 300
CAAGAAAATC GCTTCAGAAT CTTGAGTTGG AGTACCTTGA TTTGTATCTG 350
ATACACTGGC CCATCAGTGC CAAGCCTGGG AAAGTTGAGT CACGCACTAG 400
AGGGAGAAGG ACCAAATGCC GATGGACTTC AAGGGTGTGT GGGCAGACAT 450
GGAGGAAGCT CAGAGACTTG GCCTCACCAA ATCCATTGGG AATCAGCAAT 500
TTCTCTACCA AAAAGACTCA GAATTTGCTC TCCTTTGGCT ACTATTCCTC 550

CGTCAGTCAA TCAANTTTAA NATGANTCCA TTTTGGCAAC AGAAGAACCT 600
CAAAAACTTC TGCAAGGCCA GTGGTATAAT TTGTGACTGG CTTCTCCCCA 650
TTGGGTGCCA TNNGAACCAN TTGGGGGCAC CAATCATGTT CTCNA 695

(2) INFORMATION FOR SEQ ID NO:36:

(i) SEQUENCE CHARACTERISTICS:
(A) LENGTH: 765
(B) TYPE: cDNA
(C) STRANDEDNESS: Single
(D) TOPOLOGY: Linear

(ix) FEATURES
(D) OTHER INFORMATION: Protein kinase

(xi) SEQUENCE DESCRIPTION: SEQ ID:36:
GGGCANANCG TGTTGTGGGA ACTGGGTCAT TTGGAATTGT ATTCCANGCG 50
AAATGCTTGG AAACTGGTGA GACTGTGGCC ATAAAGAAGG TTTTACAGGA 100
CAGAAGGTAT AAGAACAGGG AACTTCAATT GATGCGCGTA ATGGATCATC 150
CAAATGTGAT TTGTTTGAAG CATTGTTTCT TCTCTACAAC AAGCAAAAAT 200
GAGCTTTTTC TCAATTTGGT TATGGAATAT GTTCCGGAAA CTATGTATCG 250
GGTTATAAAG CATTACAGCA ATGCAAACCA GAAAATGCCC CTTGTCTATG 300
TCAAACTTTA CATGTNCCAC ATTTTCAGAG GGCTGGCTTA CATACACACC 350
GTTCCTGGAG TTTGCCATAN ANATTTGAAN CCTCCAAATT TATTGGTTGA 400
TCCTCTTATT CACCANGTCA AGCTTTGTTG ATTTTGGAAG TGCCAAAATG 450
CNGGTGAAAG GNGAAACAAA CATANCATAC CTATGTTTCA CGTTTCTATC 500
NGGCTCCNCG AAACTAATTT TTTGGTGCCN CCNGATTATA CCACTTCCCA 550
TTGATATCTG GTCNGCTGGC TGTGTCCTAA NCAAAACTTC CTTTTGGGCC 600
CCCCTTTGTT TCCCTGGAAA AAAATGCCAT NGAACCACCT GTTAAAAATC 650

NTTCCNGGTT CNGGGGAACA CCNCNCCNTT CAAAAAATCC CCNTTTTGAA 700
TCCCCANTTN TACCAAATTC CCGGTTTCCN CCGAAAAAAN CCCNCCCTTT 750
GGNNNAAGGT TTTCC 765

(2) INFORMATION FOR SEQ ID NO:37:

(i) SEQUENCE CHARACTERISTICS:
(A) LENGTH: 772
(B) TYPE: cDNA
(C) STRANDEDNESS: Single
(D) TOPOLOGY: Linear

(ix) FEATURES
(D) OTHER INFORMATION: Auxin-related gene

(xi) SEQUENCE DESCRIPTION: SEQ ID:37:
GGAGAAACCT CTGCCCTTTA AACTTTACTT TTGCAATACA CCGTCTAACA 50
ATGGCTGCAG CTCCAAGTGA GTCCATACCC TCTGTAAATA AGGCCTGGGT 100
CTATTCAGAG TATGGAAAAA CTGCTGATGT TCTCAAGTTT GATCCAAGTG 150
TGGCTGTTCC TGAAATTAAA GAGGATCAGG TGCTGATCAA GGTTGTTGCT 200
GCTTCTCTTA ACCCAGTTGA TTTTAAGAGG GCTCTTGGTT ACTTCAAGGA 250
CACTGACTCT CCCCTACCTA CAATTCCAGG GTATGATGTA GCTGGTGTGG 300
TGGTAAAGGT AGGAAGCCAA GTAACCAAGT TTAAGGTGGG GGATGAAGTG 350
TATGGGGATC TCAATGAAGA CAGCATTGGT GAAACCCAAC AAGGTTTGGG 400
TCTTTGGCAG AGTACACTGC TGCAGATGAA AGANTATTGG CTCACAAACC 450
CAAAAACCTG AGCTTTATTG AAGCTGCTAA CCTTCCCTTG GCTATTGAAA 500
CTGCCCATGA AGGGCTTGAA AGAACTGAAC TTTCTGCTGG TAAATCCGTC 550
CTTGTTTTGG GAAGCGCTGG GGGTNTTGGA ACACATATTA TCANCTTGCC 600
AAAGCATGTT TTTGGTGCTT CCCAANTAAC NNCTACTGCA ANCACTAAAA 650

AACCGGAATT TGTTGAAAAA CCTGGGTNCT GATTTGGCTA CCAATTACCC 700
CANGAAAACT TCCAAGAACT GCCCAAAAAA TTGAATTTTN TTTTTNANGC 750
CNTTNGGGAA ANNAANAAGG GT 772

(2) INFORMATION FOR SEQ ID NO:38:

(i) SEQUENCE CHARACTERISTICS:
(A) LENGTH: 773
(B) TYPE: cDNA
(C) STRANDEDNESS: Single
(D) TOPOLOGY: Linear

(ix) FEATURES
(D) OTHER INFORMATION: Sucrose transporter

(xi) SEQUENCE DESCRIPTION: SEQ ID:38:
CATTGGCGAT CACGTACAGT GTTCCATATG CCTTGATTTC TTCTCGTATC 50
GAGTCTTTGG GACTTGGCCA AGGCTTATCA ATGGGTGTAC TGAATCTGGC 100
AATCGTAGTA CCACAGGTGC TGGTATCCCT GGGAAGTGGA CCATGGGATC 150
AGCTATTTGG TGGTGGAAAC TCTCCAGCCT TTGCGGTTGC AGCAGTTGCA 200
GCCTTAGCAA GTGGGCTGGT GGCCATCTTG GCTATTCCAC GTTCTATTCC 250
ACAGAAGCCT ANATCTTTCA CATGAGGTAT TTTGTTGTAT CTACTTTTTA 300
CCCAACTTTG TCACAGAAAT ACAAAACCTC CATAGATAGT GAGAATTTGT 350
AAATATCTTT TGTTACGTGT TAGCTATTTC TCAATACACT CATTTACCAG 400
AGGTTTCTTT AGTTCTGGAA ATTTCTCTCT TTCCCTTTTT GTCGTTTTAG 450
ATGCTTTAAT AAAGAAAGGC CTGGCAGCGA TTATATCAAA GTTGANCTGA 500
ATATCTGTGT TGAAGTGCTT CCGTTCAACA ATTTATAGTT CTCAATTTCT 550
ACAATATTTT AAATCAGAAC TGTCCCCTGG TTGGACCCTA ATGGAATCCA 600
TATGTTGGAA CCATAATCTC AATTANGCAT CCTGCCTCAA TTCCNCAATG 650

GTGTTTTCAN AANTGTTGAN GAAACNANTT NNTCCAAAAA GTTGATGGTG 700
TTTTTCCCAA ATGCCNGGCT ACNCCACCAA NNTTGANGTT NGGTACNCCA 750
AATTGAATNA AGTTATTACC CAC 773